(19) Europäisches Patentamt
European Patent Office
Office européen des brevets

(11) EP 1 510 524 A2

(12) EUROPEAN PATENT APPLICATION

(43) Date of publication:
02.03.2005 Bulletin 2005/09

(21) Application number: 03025811.5

(22) Date of filing: 07.06.1996

(51) Int Cl.[7]: **C07K 7/00**, C07K 7/06, C07K 7/08, C07K 7/64, C07K 9/00, C07K 14/00, A61K 38/00, A61K 38/03, A61K 38/08, A61K 38/10, C07K 14/505, C07K 7/04

(84) Designated Contracting States:
AT BE CH DE DK ES FI FR GB GR IE IT LI LU MC NL PT SE

(30) Priority: 07.06.1995 US 484631
07.06.1995 US 484635

(62) Document number(s) of the earlier application(s) in accordance with Art. 76 EPC:
96919296.2 / 0 886 648

(71) Applicants:
- ORTHO PHARMACEUTICAL CORPORATION
New Brunswick, NJ 08933-7003 (US)
- Affymax, Inc.
Palo Alto, CA 94303 (US)

(72) Inventors:
- Wrighton, Nicholas, C.
Winchester, MA 01890 (US)
- Dower, William, J.
Menlo Park, CA 94025 (US)
- Chang, Ray, S.
Colma, CA 94015 (US)
- Kashyap, Arun, K.
Newark, CA 94560-1463 (US)
- Jolliffe, Linda, K.
Bellemeade, NJ 08502 (US)
- Johnson, Dana
Upper Black Eddy, PA 18972 (US)
- Mulcahy, Linda
Yardley, PA 19067 (US)

(74) Representative:
Nunney, Ronald Frederick Adolphe et al
Hepworth Lawrence Bryer & Bizley,
Merlin House,
Falconry Court,
Baker's Lane
Epping, Essex CM16 5DQ (GB)

Remarks:
This application was filed on 10 - 11 - 2003 as a divisional application to the application mentioned under INID code 62.

# (54) Compounds and peptides that bind to the erythropoietin receptor

(57) Peptides of 10 to 40 amino acid residues in length that binds to and activates erythropoietin receptor or otherwise behaves as an erythropoietin agonist, and comprises a sequence of amino acids $X_3X_4X_5GPX_6TWX_7X_8$, optionally cyclized or dimerized, where each amino acid is indicated by standard one letter abbreviation; $X_6$ is independently selected from any one of the 20 genetically coded L-amino acids; $X_3$ is C, A, α-amino-γ-bromobutyric acid, or Hoc, where Hoc is homocysteine; $X_4$ is R, H, L, or W; $X_5$ is M, F, or I; $X_7$ is D, E, I, L, or V; and $X_8$ is C, A, α-amino-γ-bromobutyric acid, or Hoc, where hoc is homocysteine, provided that either $X_3$ or $X_8$ is C or Hoc.

EP 1 510 524 A2

## Description

This application is a continuation-in-part of 08/484,635 and 08/484,631, filed June 7, 1995, which are continuation-in-parts of Serial No. 08/155,940, filed November 19, 1993, all of which are incorporated herein by reference.

FIELD OF THE INVENTION

The present invention provides, *inter alia*, peptides and compounds that bind and activate the erythropoietin receptor (EPO-R) or otherwise act as an EPO agonist. The invention has application in the fields of biochemistry and medicinal chemistry and particularly provides EPO agonists for use in the treatment of human disease.

BACKGROUND OF THE INVENTION

Erythropoietin (EPO) is a glycoprotein hormone with 165 amino acids, 4 glycosylation sites on amino-acid positions 24, 38, 83, and 126, and a molecular weight of about 34,000. It is initially produced as a precursor protein with a signal peptide of 23 amino acids. EPO can occur in three forms: α, β, and asialo. The α and β forms differ slightly in the carbohydrate components, but have the same potency, biological activity, and molecular weight. The asialo form is an α or β form with the terminal carbohydrate (sialic acid) removed. The DNA sequences encoding EPO have been reported. *See,* Lin (1987) U.S. Patent No. 4,703,008, which is incorporated herein by reference.

EPO stimulates mitotic division and the differentiation of erythrocyte precursor cells and thus ensures the production of erythrocytes. It is produced in the kidney when hypoxic conditions prevail. During EPO-induced differentiation of erythrocyte precursor cells, there is induction of globin synthesis and increases in the synthesis of the heme complex and in the number of ferritin receptors. This makes it possible for the cell to take on more iron and synthesize functional hemoglobin. Hemoglobin in mature erythrocytes binds oxygen. Thus, the erythrocytes and the hemoglobin contained in them play a key part in supplying the body with oxygen. The complex processes which have been described are initiated by the interaction of EPO with an appropriate receptor on the cell surface of the erythrocyte precursor cells. *See, e.g.*, Graber and Krantz (1978) *Ann. Rev. Med. 29*:51-66.

EPO is present in very low concentrations in plasma when the body is in a healthy state wherein tissues receive sufficient oxygenation from the existing number of erythrocytes. This normal low concentration is enough to stimulate replacement of red blood cells which are lost normally through aging.

The amount of EPO in the circulation is increased under conditions of hypoxia when oxygen transport by blood cells in the circulation is reduced. Hypoxia may be caused by loss of large amounts of blood through hemorrhage, destruction of red blood cells by over-exposure to radiation, reduction in oxygen intake due to high altitudes or prolonged unconsciousness, or various forms of anemia. In response to tissues undergoing hypoxic stress, EPO will increase red blood cell production by stimulation of proliferation of erythroid progenitor cells. When the number of red blood cells in circulation is greater than needed for normal tissue oxygen requirements, EPO in circulation is decreased.

Because EPO is essential in the process of red blood cell formation, the hormone has potentially useful applications in both the diagnosis and the treatment of blood disorders characterized by low or defective red blood cell production. Recent studies have provided a basis for the projection of efficacy of EPO therapy in a variety of disease states, disorders, and states of hematologic irregularity, including: beta-thalassemia *(see,* Vedovato *et al.* (1984) *Acta. Haematol. 71*:211-213); cystic fibrosis *(see,* Vichinsky *et al.* (1984) *J. Pediatric 105*:15-21; pregnancy and menstrual disorders *(see,* Cotes *et al.* (193) *Brit. J. Ostet. Gyneacol. 90*:304-311; early anemia of prematurity *(see,* Haga *et al*. (1983) *Acta Pediatr. Scand. 72*; 827-831); spinal cord injury *(see,* Claus-Walker *et al*. (1984) *Arch. Phys. Med. Rehabil. 65*:370-374); space flight *(see,* Dunn *et al.* (1984) *Eur. J. Appl. Physiol. 52*:178-182); acute blood loss *(see,* Miller *et al*. (1982) *Brit. J. Haematol. 52*:545-590); aging *(see,* Udupa *et al*. (1984) *J. Lab. Clin. Med. 103*:574-580 and 581-588 and Lipschitz *et al.* (1983) *Blood 63*:502-509; various neoplastic disease states accompanied by abnormal erythropoiesis *(see,* Dainiak *et al*. (1983) *Cancer 5*:1101-1106 and Schwartz *et al.* (1983) *Otolaryngol. 109*:269-272); and renal insufficiency *(see,* Eschbach *et al.* (1987) *N. Eng. J. Med. 316*:73-78).

Purified, homogeneous EPO has been characterized. See, Hewick U.S. Patent No. 4,677,195. A DNA sequence encoding EPO was purified, cloned and expressed to produce synthetic polypeptides with the same biochemical and immunological properties. A recombinant EPO molecule with oligosaccharides identical to those on the natural material has also been produced. *See,* Sasaki *et al*. (1987) *J. Biol. Chem. 262*:12059-12076.

Despite the availability of purified recombinant EPO, little is known concerning the mechanism of EPO-induced erythroblast proliferation and differentiation. The specific interaction of EPO with progenitors of immature red blood cells, platelets, and megakaryocytes remains to be characterized. This is due, at least in part, to the small number of surface EPO receptor molecules on normal erythroblasts and on the erythroleukemia cell line. *See,* Krantz and Goldwasser (1984) *Proc. Natl. Acad. Sci. USA 81*:7574-7578; Branch *et al.* (1987) *Blood 69*:1782-1785; Mayeux *et al.* (1987) *FEBS Letters 211*:229-233; Mufson and Gesner (1987) *Blood 69*:1485-1490; Sakaguchi *et al*. (1987) *Biochem.*

*Biophys. Res. Commun. 146*:7-12; Sawyer *et al.* (1987) *Proc. Notl. Acad. Sci. USA 84*:3690-3694; Sawyer *et al.* (1987) *J. Biol. Chem. 262*:5554-5562; and Todokoro *et al.* (1988) *Proc. Natl. Acad. Sci. USA 84*:4126-4130.

Cross-linked complexes between radioiodinated EPO and cell surface proteins suggest that the cell surface proteins comprise two polypeptides having approximate molecular weights of 85,000 daltons and 100,000 daltons, respectively. More recently, the two cross-linked complexes have been subjected to V8 protease digestion and have been found to have identical peptide fragments, suggesting that the two EPO-binding polypeptides may be products of the same or very similar genes. *See,* Sawyer *et al.* (1988) *supra*. Most cell surface binding studies, however, have revealed a single class of binding sites, averaging 300 to 600 per cell surface, with a Kd of approximately 800 pM (picomolar). *See,* Sawyer *et al.* (1987) *Proc. Natl. Acad. Sci. USA 84*:3690-3694. However, EPO-responsive splenic erythroblasts, prepared from mice injected with the anemic strain (FVA) of the Friend leukemia virus, demonstrate a high and a low affinity binding site with dissociation constants of 100 pM and 800 pM, respectively. *See,* Sawyer *et al.* (1987) *J. Biol. Chem. 262*:5554-5562 and Landschulz (1989) *Blood* 73:1476-1478. The DNA sequences and encoded peptide sequences for murine and human EPO receptor proteins have been described. *See*, D'Andrea *et al.* PCT Patent Publication No. WO 90/08822 (published 1990).

The availability of cloned genes for the EPO-R facilitates the search for agonists and antagonists of this important receptor. The availability of the recombinant receptor protein allows the study of receptor-ligand interaction in a variety of random and semi-random peptide diversity generation systems. These systems include the "peptides on plasmids" system described in U.S. patent application Serial No. 778,233, filed October 16, 1991, the "peptides on phage" system described in U.S. patent application Serial No. 718,577, filed June 20, 1991, and in Cwirla *et al*., Aug. 1990, *Proc. Natl. Acad. Sci. USA 87*:6378-6382, the "encoded synthetic library" (ESL) system described in U.S. patent application Serial No. 946,239, filed September 16, 1992, which is a continuation-in-part application of Serial No. 762,522, filed September 18, 1991, and the "very large scale immobilized polymer synthesis" system described in U. S. patent application Serial No. 492,462, filed March 7, 1990; PCT patent publication No. 90/15070, published December 13, 1990; U.S. patent application Serial No. 624,120, filed December 6, 1990; Fodor *et al*., 15 Feb. 1991, *Science 251*:767-773; Dower and Fodor, 1991, *Ann. Rep. Med. Chem. 26*:271-180; and U.S. patent application Serial No. 805,727, filed December 6, 1991; each of the foregoing patent applications and publications is incorporated herein by reference.

There remains a need, however, for compounds that bind to or otherwise interact with the EPO-R, both for studies of the important biological activities mediated by this receptor and for treatment of disease. The present invention provides such compounds.

## SUMMARY OF THE INVENTION

In one embodiment, the invention provides peptides that bind to and activate the EPO-R or otherwise behave as EPO agonists. These peptides are 10 to 40 or more amino acid residues in length, preferably 14 to 20 amino acid residues in length, and comprise a core sequence of amino acids $X_3X_4X_5GPX_6TWX_7X_8$ (SEQ ID NO:), where each amino acid is indicated by standard one letter abbreviation; $X_3$ can be C, A, α-amino-γ-bromobutyric acid, or Hoc, where Hoc is homocysteine; $X_4$ can be R, H, L, or W; $X_5$ can be M, F, or I; $X_6$ is independently selected from any one of the 20 genetically coded L-amino acids or the stereoisomeric D-amino acids; $X_7$ can be D, E, I, L, or V; and $X_8$ can be C, A, α-amino-γ-bromobutyric acid, or Hoc, where Hoc is homocysteine, provided that either $X_3$ or $X_8$ is C or Hoc. Preferably, the peptide will comprise a core sequence $YX_2X_3X_4X_5GPX_6TWX_7X_8$ (SEQ ID NO:), where each amino acid is indicated by standard one letter abbreviation; each $X_2$ and $X_6$ is independently selected from any one of the 20 genetically coded L-amino acids; $X_3$ can be C, A, α-amino-γ-bromobutyric acid, or Hoc, where Hoc is homocysteine; $X_4$ can be R, H, L, or W; $X_5$ can be M, F, or I; $X_7$ can be D, E, I, L, or V; and $X_8$ can be C, A, α-amino-γ-bromobutyric acid, or Hoc, where Hoc is homocysteine, provided that either $X_3$ or $X_8$ is C or Hoc.

More preferably, the peptide will comprise a core sequence of amino acids $X_1YX_2X_3X_4X_5GPX_6TWX_7X_8X_9X_{10}X_{11}$ (SEQ ID NO:), where each amino acid is indicated by standard one letter abbreviation; each $X_1$ $X_2$, $X_6$, $X_9$, $X_{10}$, and $X_{11}$ is independently selected from any one of the 20 genetically coded L-amino acids; $X_3$ can be C, A, α-amino-γ-bromobutyric acid, or Hoc, where Hoc is homocysteine; $X_4$ can be R, H, L, or W; $X_5$ can be M, F, or I; $X_7$ can be D, E, I, L, or V; and $X_8$ can be C, A, α-amino-γ-bromobutyric acid, or Hoc, where Hoc is homocysteine, provided that either $X_3$ or $X_8$ is C or Hoc.

In a more preferred embodiment, both $X_3$ and $X_8$ will be C and, thus, the peptide will comprise a core sequence of amino acids $X_1YX_2CX_4X_5GPX_6TWX_7CX_9X_{10}X_{11}$ (SEQ ID NO:). More preferably, the peptide will comprise a core sequence of amino acids $X_1YX_2CX_4X_5GPX_6TWX_7CX_9X_{10}X_{11}$ (SEQ ID NO:), where $X_4$ can be R or H; $X_5$ can be F or M; $X_6$ can be I, L, T, M, or V; $X_7$ is D or V; $X_9$ can be G, K, L, Q, R, S, or T; and $X_{10}$ can be A, G, P, R, or Y. In a most preferred embodiment, the peptide will comprise a core sequence of amino acids $X_1YX_2CX_4X_5GPX_6TWX_7CX_9X_{10}X_{11}$ (SEQ ID NO:), where X, can be D, E, L, N, S, T, or V; $X_2$ can be A, H, K, L, M, S, or T; $X_4$ is R or H; $X_9$ can be K, R, S, or T; and $X_{10}$ is P. Particularly preferred peptides include but are not limited to, the following:

GGLYLCRFGPVTWDCGYKGG;
GGTYSCHFGPLTWVCKPQGG;
GGTYSCHFGPLTWVCKPQ;
GGDYHCRMGPLTWVCKPLGG;
VGNYMCHFGPITWVCRPGGG;
GGNYMCHFGPITWVCRPGGG;
GGVYACRMGPITWVCSPLGG;
VGNYMAHMGPITWVCRPGG;

GGPHHVYACRMGPLTWIC;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
SCHFGPLTWVCK;
GGTYSCFGPLTWVCKPQGG;
TYSCHFGPLTWVCKPQGG;
YSCHFGPLTWVC;
GGTYSCHFGPLTFVCKPQGG; and
HFGPLTWV.

In another preferred embodiment, the peptides of the present invention are cyclized or dimerized. Examples of peptides which can be cyclized as C-terminal amides include, but are not limited to, the following:

GGTYSCHFGPLTWVCKPQGG;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;

GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
YSCHFGALTWVCK;
SCHFGPLTWVCK;
GGTYSEHFGPLTWVKKPQGG;
GGTYSCFGPLTWVCKPQGG;
TYSCHFGPLTWVCKPQGG;
YSCHFGPLTWVC; and
GGTYSCHFGPLTFVCKPQGG.

An example of a dimer in accordance with the present invention follows:

```
GGTYSCHFGPLTWVCKPQGG
     |         |
GGTYSCHFGPLTWVCKPQGG.
```

According to some embodiments of this invention, two or more, and preferably between two to six amino acid residues, independently selected from any of the 20 genetically coded L-amino acids or the stereoisomeric D-amino acids, will be coupled to either or both ends of the core sequences described above. For example, the sequence GG will often be appended to either or both termini of the core sequences for ease in synthesis of the peptides. The present invention also provides conjugates of these peptides and derivatives and peptidomimetics of the peptides that retain the property of EPO-R binding.

The present invention also provides methods for treating disease involving a deficiency of EPO utilizing the novel compounds of the invention. The present invention further provides pharmaceutical compositions comprising one or more compounds of the invention and a physiologically acceptable carrier.

BRIEF DESCRIPTION OF THE DRAWINGS

**Figure 1** provides the sequences of the mutagenesis oligomers employed in the procedures described herein.
**Figure 2** provides the sequences of representative peptides of this invention.
**Figure 3** illustrates a new phagemid mutagenesis library constructed using the pVIII display system. In this library, the tyrosine, glycine-proline and threoninetryptophan residues (underlined) were fixed, as well at the two cysteines. Other positions between the cysteine residues (shown in outline italic typeface present in the original AF11154 hit peptide) were mutated by oligo construction such that each amino acid residue could change to any other at a frequency of 50%. "X" denotes a random amino acid position.
**Figures 4A-C** illustrate pIII and pVIII phagemid mutagenesis libraries currently under construction and screening. Amino acid residues in bold face are fixed, the remainder (indicated by NNK) are randomized. 4A (ON3007) and 4C (ON3017) are designed to investigate the contribution of extra flanking regions N-terminal and C-terminal, respectively, to the core sequence (tyrosine to the second cysteine). 4B (ON3016) is based on peptide Y-CHFGPLTWVC, where the tyrosine residue is placed directly next to the first cysteine. This library adds additional random residues on both sides of the core sequence.
**Figure 5** illustrates a mutagenesis library, based on GGTYSCHFGPLTWVCKPQGG, constructed and screened using a new Lac-I display vector ("Headpiece Dimer"). Amino acid residues denoted X are random (NNK), those underlined are lightly mutated (91:3:3:3/91:3:3:3/K), whereas those in outline are mutated more heavily (70:10:10/70:10:10/K).
**Figure 6** illustrates a mutagenesis library, based on TIAQYICYMGPETWECRPSPKA, constructed and screened using a new Lac-I display vector ("Headpiece Dimer"). Amino acid residues denoted X are random (NNK), those in outline were fixed, and the two glutamic acid residues were lightly mutated (91:3:3:3/91:3:3:3/K).
**Figure 7** is a graphical depiction of the results of the FDCP-1/hEPO-R bioassay for selected peptides of the invention with:

• designating the results for GGCRIGPITWVCGG;
o designating the results for GGLYLCRFGPVTWDCGYKGG;
■ designating the results for GGTYSCHFGPLTWVCKPQGG;
▲ designating the results for GGDYHCRMGPLTWVCKPLGG;
▼ designating the results for VGNYMCHFGPITWVCRPGGG;
ı designating the results for GGVYACRMGPITWVCSPLGG;
+ designating the results for VGNYMAHMGPITWVCRPGG; and
* designating the results for EPO.

**Figure 8** is a graphical depiction of the results of the TF-1 bioassay for EPO (▲ and ♦ designate the results for the assay using EPO and 10,000 or 20,000 cells per well, respectively); and for the peptide VGNYMCHFGPITWVCRPGGG (SEQ ID NO:) (■ and • designate the results for the assay using the peptide and 10,000 or 20,000 cells per well, respectively).
**Figures 9A** (control), **9B** (treated with 500 pM EPO) and **9C** (treated with 25 mm GGDYHCRMGPLTWVCKPLGG) are photographs depicting the results of a MTT proliferation assay on representative populations of spleen cells of phenylhydrazine treated mice at 200 X magnification.
**Figure 10** is a graphical depiction of the results of the FDCP-1/hEPOR bioassay showing increased potency of the biotinylated peptide (*i.e.*, GGTYSCHFGPLTWVCKPQGGSSK (Ahx-biotin)), by oligomerization with streptavidin. Peptides GGTYSCHFGPLTWVCKPQGG and GGTYSCHFGPLTWVCKPQGGSSK (Ahx-biotin) exhibit approximately the same activity, but when the latter is pre-complexed with streptavidin (*i.e.*, GGTYSCHFGPLTWVCKPQGGSSK (Ahx-biotin)-streptavidin complex), it is far more potent. In this assay, little reduction in activity is seen at the lowest concentration (5 nM with respect to peptide in the complex). Streptavidin, alone, has marginal activity at high concentrations.
**Figure 11** is a graphical depiction of the results of the FDCP-1/hEPOR bioassay showing polyclonal goat anti-biotin-mediated increase in potency of peptide AF11505. Pre-complexing GGTYSCHFGPLTWVCKPQGGSSK (Ahx-biotin) with goat anti-biotin antibodies (GGTYSCHFGPLTWVCKPQGGSSK + G anti-B) increases the potency of the peptide one log over the free peptide. The purified antibodies (G anti-B) have no stimulatory effect alone.
**Figure 12** illustrates the synthetic scheme for the preparation of the dimeric peptide analog of GGTYSCHFGPLTWVCKPQGG.
**Figure 13** illustrates the $IC_{50}$ plot of the dimeric peptide analog of GGTYSCHFGPLTWVCKPQGG. Affinity was determined using a radioligand competition binding assay against PIG-tailed EPOR immobilized on mAB179.

**Figure 14** illustrates the *in vitro* biological activity of the dimeric peptide analog of GGTYSCHFGPLTWVCKPQGG using the FDCP-1/hEPOR cell proliferation assay. The dimeric peptide has an $EC_{50}$ of approximately 20 nM, a 20-fold increase in potency over the parental peptide.

**Figure 15** illustrates the cell cycle progression of FDC-P1/ER.

**Figure 16** illustrates the equilibrium binding analysis of the specific association of [$^{125}$I]EPO with EBP immobilized on agarose beads and indicates a Kd of 5 nM ± 2 based on a linear transformation (Scatchard) of the binding isotherm.

**Figures 17A, 17B** and **17C** illustrate the results of the polycythemic exhypoxic mouse bioassay using peptides GGTYSCHFGPLTWVCKPQGG, GGTYSCHFGPLTWVCKPQ and LGRKYSCHFGPLTWVCQPAKKD, respectively.

**Figures 18A** and **18B** illustrate the results of the polycythemic exhypoxic mouse bioassay using peptides TIAQYICYMGPETWFCRPSPKA and a dimeric peptide analog of GGTYSCHFGPLTWVCKPQGG containing two disulfide bonds (AF12080), respectively.

**Figures 19A** and **19B** illustrate the results of the reticulocyte assay using EPO and GGTYSCHFGPLTWVCKPQGG, respectively. For both dose groups, n = 10 animals. EPO: 0.4, 4.0, 40.0 Units/mouse, total dose vehicle control; GGTYSCHFGPLTWVCKPQGG: 2.0, 20.0, 200.0 μg/mouse, total dose vehicle control + DMSO (1-2 %) 200 μg/mouse = 10 mg/kg. Rational: *in vitro* proliferation assay data suggests: 2 μg of GGTYSCHFGPLTWVCKPQGG = 0.4 Units of EPO.

**Figures 20A** and **20B** illustrate the results of the reticulocyte assay using EPO and GGTYSCHFGPLTWVCKPQGG, respectively. For both dose groups, n = 10 animals. EPO : 0.4, 4.0, 40.0 units/mouse, total dose; GGTYSCHFGPLTWVCKPQGG: increase dose to 1 mg/mouse, total dose = 50 mg/kg and to 2 mg/mouse, total dose = mg/kg.

DETAILED DESCRIPTION OF THE INVENTION AND PREFERRED EMBODIMENT

The following terms are intended to have the following general meanings:

Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

Stereoisomers (*e.g.*, D-amino acids) of the twenty conventional amino acids, unnatural amino acids such as a,a-disubstituted amino acids, N-alkyl amino acids, lactic acid, and other unconventional amino acids may also be suitable components for compounds of the present invention. Examples of unconventional amino acids include: 4-hydroxyproline, O-phosphoserine, N-acetylserine, N-formylmethionine, 3-methylhistidine, 5-hydroxylysine, and other similar amino acids and imino acids (*e.g.*, 4-hydroxyproline).

"Agonist" refers to a biologically active ligand which binds to its complementary biologically active receptor and activates the latter either to cause a biological response in the receptor, or to enhance preexisting biological activity of the receptor.

"Host Cell" refers to a eukaryotic or procaryotic cell or group of cells that can be or has been transformed by a recombinant DNA vector. For purposes of the present invention, procaryotic host cells are preferred.

"Peptide" or "polypeptide" refers to a polymer in which the monomers are alpha amino acids joined together through amide bonds. Peptides are two or often more amino acid monomers long.

"Pharmaceutically acceptable salts" refers to the non-toxic alkali metal, alkaline earth metal, and ammonium salts commonly used in the pharmaceutical industry including the sodium, potassium, lithium, calcium, magnesium, barium, ammonium and protamine zinc salts, which are prepared by methods well known in the art. The term also includes non-toxic acid addition salts, which are generally prepared by reacting the compounds of this invention with a suitable organic or inorganic acid. Representative salts include the hydrochloride, hydrobromide, sulfate, bisulfate, acetate, oxalate, valerate, oleate, laurate, borate, benzoate, lactate, phosphate, tosylate, citrate, maleate, fumarate, succinate, tartrate, napsylate and the like.

"Pharmaceutically or therapeutically effective dose or amount" refers to a dosage level sufficient to induce a desired biological result. That result can be alleviation of the signs, symptoms, or causes of a disease, or any other desired alteration of a biological system. Preferably, this dose or amount will be sufficient to stimulate the EPO-R and, thus, alleviate the symptoms associated with a deficiency of EPO, or a defective or low red blood population *in vivo*,.

"Recombinant DNA Cloning or Expression Vector" refers to a DNA or RNA molecule that encodes a useful function and can be used to transform a host cell. For purposes of the present invention, a cloning vector typically serves primarily as an intermediate in the construction of an expression vector; the latter vector is used to transform or transfect a host cell so that the transformed host cell produces a protein or other product encoded by the vector.

Such vectors are typically "plasmids," which for purposes of the present invention, are vectors that can be extrachromosomally maintained in a host cell, but can also be vectors that integrate into the genome of a host cell. Those of skill in the art may refer to "cloning vectors", as defined herein, as "vectors" and to "expression vectors, " as defined herein," as "plasmids."

The present invention provides compounds that bind to and activate the EPO-R or otherwise behave as an EPO agonist. These compounds include "lead" peptide compounds, discovered with random peptide diversity generating systems, and "derivative" compounds constructed so as to have the same or similar molecular structure or shape as the lead compounds, but that differ from the lead compounds either with respect to susceptibility to hydrolysis or proteolysis and/or with respect to other biological properties, such as increased affinity for the receptor, increased *in vitro* activity or *in vivo* activity.

The random peptide diversity generating systems initially employed included the "peptides on phage" system discussed above. The random peptides were designed to be 8 amino acid residues in length, flanked by Cys residues at both termini (thus, 10 amino acid residues in total length). In these initial systems, the random peptides were presented as part of a fusion protein comprising the pVIII coat protein of a phage fd derivative (peptides on phage). The fusion proteins, along with the DNA encoding the fusion proteins, were "panned" on immobilized EPO-R. The panning process involved multiple rounds of incubating the fusion proteins with the immobilized receptor, collecting the fusion proteins that bound to the receptor (along with the accompanying DNA), and producing more of the fusion proteins collected.

Typically, after three rounds of panning, the fusion proteins and accompanying DNA were isolated and cultured to produce fusion protein preparations for an ELISA to determine if the fusion protein bound specifically to the receptor. This assay was carried out similarly to the panning, except that after removing unbound fusion proteins, the wells were treated with rabbit anti-phage antibody (or with anti-lac antibody for the peptides on plasmids system), then with alkaline phosphatase-conjugated goat anti-rabbit antibody, and then the amount of alkaline phosphatase in each well was determined by standard methods. By comparing test wells with control wells (no receptor), one can determine whether the fusion proteins bound to the receptor specifically.

The immobilized receptor used in the panning and ELISA procedures comprised the extracellular domain of the EPO-receptor and was produced in recombinant host cells. This receptor molecule can be produced in a variety of different forms and host cells. One useful form is constructed with a signal peptide for protein secretion and for glycophospholipid membrane anchor attachment (this form of anchor attachment is called "PIG-tailing;" *see,* Caras and Weddell, 3 March 1989, *Science 243*:1196-1198; and Lin *et al*., 10 Aug. 1990, *Science 249*:677-679, each of which is incorporated herein by reference). This system allows for cleavage of the receptor from the surface of the cells expressing the receptor and collection of the cleaved receptor quite easily. Preferably, a protease cleavage site, such as a thrombin cleavage site, is inserted between the HPAP epitope of the PIG-tailed receptor and the receptor itself.

The recombinant receptor protein was immobilized using the following methodology. Microtiter plates were coated within an anti-receptor antibody and the wells which were to contain the immobilized receptor were treated with bovine serum albumin (BSA) to block non-specific binding. The PIG-tailed receptor having a thrombin cleavage site was added to the coated wells of the microtiter plate, which were then washed to remove unbound receptor.

When using random peptide generation systems that allow for multivalent ligand-receptor interaction, one must recognize that the density of the immobilized receptor is an important factor in determining the affinity of the ligands that will bind to the immobilized receptor. At higher receptor densities (*i.e.*, each anti-receptor antibody-coated well treated with 0.25 to 0.5 mg of receptor), multivalent binding is more likely to occur (if at all) than at lower receptor densities (*i.e.*, each anti-receptor antibody-coated well treated with 0.5 to 1 ng of the receptor). If multivalent binding is occurring, then one will be more likely to isolate ligands with relatively low affinity. Typically, one can identify lead compounds using a high density of immobilized receptor and then test the derivatives of the lead compound at lower receptor densities to isolate compounds with higher affinity for the receptor than the lead compound.

Often, the receptor was added only to alternate rows of the microtiter plate; the BSA-blocked wells in the "blank" rows served as useful negative controls to determine whether a receptor-specific reaction was creating the observed results. Fusion protein preparations were then added to the wells and incubated to allow binding to the receptor to occur; then, the wells were washed to remove unbound fusion proteins.

With the above systems, a peptide was discovered that bound to the EPO-R. The DNA encoding the fusion protein that bound to the receptor was sequenced. This peptide had the sequence: GGCRIGPITWVCGG (SEQ ID, NO:) (the N-terminal GG residue allowing for cleavage on the phage). This peptide consistently showed low affinities to BSA and to the anti-receptor antibody and high affinity to EPO-R by ELISA. Moreover, the phagemid clone was competed by free EPO and by cognate free peptide. Further, the free peptide was found to compete EPO-phagemid, LacI-EPO fusion, and radioligand. Finally, the free peptide did not compete in IL-2R$\alpha\beta$ binding assays.

A mutagenesis study on this preferred peptide was then conducted. To generate the collection of oligonucleotides that encode the random peptides, the mutagenesis oligomers shown in Figure 1 were prepared where N was nucleotide A, C, G, or T (equimolar; depending on the methodology employed, other nucleotides can be employed)

and K was G or T (equimolar) in the codon motif (NNK). Those of skill in the art will recognize that the NNK motif encodes all of the amino acids, encodes only one stop codon, and reduces codon bias. There are 32 possible codons resulting from the NNK motif: 1 for each of 12 amino acids, 2 for each of 5 amino acids, 3 for each of 3 amino acids, and only one of the three stop codons.

The mutagenesis fusion proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. Preferred peptides resulting from this study are shown in Figure 2. These peptides are characterized by the motif "$X_3X_4X_5GPX_6TWX_7X_8$" (SEQ ID NO:), where each amino acid is indicated by standard one letter abbreviation; $X_6$ is independently selected from any one of the 20 genetically coded L-amino acids or the stereoisomeric D-amino acids; $X_3$ can be C, A, α-amino-γ-bromobutyric acid, or Hoc, where Hoc is homocysteine; $X_4$ can be R, H, L, or W; $X_5$ can be M, F, or I; $X_7$ can be D, E, I, L, or V; and $X_8$ can be C, A, α-amino-γ-bromobutyric acid, or Hoc, where Hoc is homocysteine, provided that either $X_3$ or $X_8$ is C or Hoc.

To ascertain a rough indication of the affinity of the peptides, the phage libraries were also screened using an affinity selection protocol wherein the peptides are competed with EPO (100 nM). This process is repeated typically for two rounds of panning. In subsequent rounds of panning, the competition temperature (4°C to ambient temperature) and time (15 to 30 minutes) as well as the temperature (4°C to ambient temperature) of the wash solutions can be altered to further select for peptides with high affinity. Using this affinity selection procedure, peptides sharing the common motif "$X_1YX_2CX_4X_5GPX_6TWX_7CX_9X_{10}X_{11}$. (SEQ ID NO:), where each amino acid is indicated by standard one letter abbreviation; each $X_1$, $X_2$, $X_6$, $X_9$, $X_{10}$, and $X_{11}$ is independently selected from any one of the 20 genetically coded L-amino acids or the stereoisomeric D-amino acids; $X_4$ can be R, H, L, or W, $X_5$ can be M, F, or I, and $X_7$ can be D, E, I, L, or V, and were isolated. In a more preferred embodiment, the peptide will comprise a sequence of amino acids $X_1YX_2CX_4X_5GPX_6TWX_7CX_9X_{10}X_{11}$ (SEQ ID NO:), where $X_4$ can be R or H, $X_5$ can be F or M, $X_6$ can be I, L, T, M, or V, $X_7$ is D or V, $X_9$ can be G, K, L, Q, R, S, or T, and $X_{10}$ can be A, G, P, R, or Y. In a most preferred embodiment, the core peptide will comprise a sequence of amino acids $X_1YX_2CX_4X_5GPX_6TWX_7CX_9X_{10}X_{11}$ (SEQ ID NO:), where $X_1$ can be D, E, L, N, S, T, or V; $X_2$ can be A, H, K, L, M, S, or T; $X_4$ is R or H; $X_9$ can be K, R, S, or T; and $X_{10}$ is P.

Examples of representative peptides falling within this motif and isolated during the affinity selection process are shown in the tables below.

# Table 1

## Peptides from Affinity Selection Protocol

### Competition with 100 nM EPO, 15 min, 4°C

| | |
|---|---|
| GGWVTCRMGPITWVCGVHGG | (SEQ ID NO:) |
| GGQLLCGIGPITWVCRWVGG | (SEQ ID NO:) |
| GGLYLCRMGPVTWECQPRGG | (SEQ ID NO:) |
| GGKYSCFMGPTTWVCSPVGRGV | (SEQ ID NO:) |
| GGWVYCRIGPITWVCDTNGG | (SEQ ID NO:) |
| GGIYKCLMGPLTWVCTPDGG | (SEQ ID NO:) |
| GGMYYCRMGPMTWVCKGAGG | (SEQ ID NO:) |

### No Competition

| | |
|---|---|
| GGTTQCWIGPITWVCRARGG | (SEQ ID NO:) |
| GGPYHCRMGPITWVCGPVGG | (SEQ ID NO:) |
| GGEYLCRMGPITWVCERYGG | (SEQ ID NO:) |
| GGEYRCRMGPISWVCSPQGG | (SEQ ID NO:) |
| GGNYTCRFGPLTWECTPQGGGA | (SEQ ID NO:) |
| GGNYVCRMGPITWICTPAGG | (SEQ ID NO:) |

## Table 2

### Peptides from Affinity Selection Protocol

### Second Competition with 100 nM EPO, 15 min, 4°C

| | |
|---|---|
| GGSWDCRIGPITWVCKWSGG | (SEQ ID NO:) |
| GGDYTCRMGPMTWICTATRG | (SEQ ID NO:) |
| GGDYNCRFGPLTWVCKPSGG | (SEQ ID NO:) |
| GGSYLCRMGPTTWLCTAQRG | (SEQ ID NO:) |
| GGDYHCRMGPLTWVCKPLGG | (SEQ ID NO:) |
| VGNYMCHFGPITWVCRPGGG | (SEQ ID NO:) |
| GGLYLCRMGPQTWMCQPGGG | (SEQ ID NO:) |
| GGTYSCHFGPLTWVCKPQGG | (SEQ ID NO:) |
| GGDYVCRMGPMTWVCAPYGR | (SEQ ID NO:) |

| | |
|---|---|
| GGLYECRMGPMTWVCRPGGG | (SEQ ID NO:) |
| GGWYSCLMGPMTWVCKAHRG | (SEQ ID NO:) |
| GGKYYCWMGPMTWVCSPAGG | (SEQ ID NO:) |

### No Competition

| | |
|---|---|
| GGYVMCRIGPITWVCDIPGG | (SEQ ID NO:) |
| GSCLQCCIGPITWVCRHAGG | (SEQ ID NO:) |
| GGNYFCRMGPITWVCQRSVG | (SEQ ID NO:) |
| GGEYICRMGPLTWECKRTGG | (SEQ ID NO:) |
| GGLYACRMGPITWVCKYMAG | (SEQ ID NO:) |
| GGQYLCTFGPITWLCRGAGGGS | (SEQ ID NO:) |
| GGVYACRMGPITWVCSPLGG | (SEQ ID NO:) |
| GGYTTCRMGPITWVCSAHGG | (SEQ ID NO:) |

## Table 3

### Peptides from Affinity Selection Protocol

#### Competition with 100 nM EPO, 15 min, Room Temp.

| | |
|---|---|
| VGNYMCHFGPITWVCRPGGG | (SEQ ID NO:) |
| GGTYKCWMGPMTWVCRPVGG | (SEQ ID NO:) |
| GGTYSCHFGPLTWVCKPQGG | (SEQ ID NO:) |
| GGDYHCRMGPLTWVCKPLGG | (SEQ ID NO:) |
| GGNYYCRFGPITFECHPTGG | (SEQ ID NO:) |
| GGLYACHMGPMTWVCQPLRGGGS | (SEQ ID NO:) |
| GGEYKCYMGPITWVCKPEGG | (SEQ ID NO:) |
| GGDYVCRMGPMTWVCAPYGRGGS | (SEQ ID NO:) |

#### No Competition

| | |
|---|---|
| GGNYVCRMGPITWICTPAGG | (SEQ ID NO:) |
| GGDYTCRMGPMTWICTATRG | (SEQ ID NO:) |
| GGEYLCRMGPMTWVCTPVGG | (SEQ ID NO:) |
| GGSYLCRMGPTTWLCTAQRGGGN | (SEQ ID NO:) |
| GGLYTCRMGPITWVCLPAGG | (SEQ ID NO:) |
| GGLYKCRMGPMTWVCSPFGG | (SEQ ID NO:) |

## Table 4

### Peptides from Affinity Selection Protocol

#### Competition with 100 nM EPO, 30 min, Room Temp.

| | |
|---|---|
| GGTYSCHFGPLTWVCKPQGG | (SEQ ID NO:) |
| GGDYHCRMGPLTWVCKPLGG | (SEQ ID NO:) |
| VGNYMCHFGPITWVCRPGGG | (SEQ ID NO:) |

#### No Competition

| | |
|---|---|
| GGDYVCRMGPMTWVCAPYGR | (SEQ ID NO:) |
| GGDYNCRFGPLTWVCKPSGG | (SEQ ID NO:) |

Another mutagenesis library having six random amino acid residues adjacent to a random 8-mer flanked by two cysteine residues was also screened against immobilized EPO-R. The mutagenesis fusion proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. The preferred peptide resulting from this study had the sequence GGPHHVYACRMGPLTWIC (SEQ ID NO:) and, thus, is encompassed by the core sequence "$YX_2X_3X_4X_5GPX_6TWX_7X_8$" (SEQ ID NO:), where each amino acid is indicated by standard one letter abbreviation; each $X_2$ and $X_6$ is independently selected from any one of the 20 genetically coded L-amino acids or the stereoisomeric D-amino acids; $X_3$ can be C, A, $\alpha$-amino-$\gamma$-bromobutyric acid, or Hoc, where Hoc is homocysteine; $X_4$ can be R, H, L, or W; $X_5$ can be M, F, or I; $X_7$ can be D, E, I, L, or V; and $X_8$ can be C, A, $\alpha$-amino-$\gamma$-bromobutyric acid, or Hoc, where Hoc is homocysteine, provided that either $X_3$ or $X_8$ is C or Hoc, wherein the core sequence is coupled at its amino terminus to a six amino acid unit, wherein each amino acid is independently selected from any of the 20 genetically coded L-amino acids or the stereoisomeric D-amino acids.

$IC_{50}$s were calculated for several of the peptides falling within the general motifs described above. These values were determined using the free peptide and are shown in Table 5 below. (Each of these peptides were independently synthesized and are C-terminally amidated, but could be easily prepared as the free carboxy acid or as an ester or other carboxy amide.)

**Table 5**

| Peptide | | $IC_{50}$ |
|---|---|---|
| GGLYLCRFGPVTWDCGYKGG | (SEQ ID NO:) | 1.67 μM |
| GGTYSCHFGPLTWVCKPQGG | (SEQ ID NO:) | 237 nm |
| GGDYHCRMGPLTWVCKPLGG | (SEQ ID NO:) | 179 nm |
| VGNYMCHFGPITWVCRPGGG | (SEQ ID NO:) | 420 nm |
| GGVYACRMGPITWVCSPLGG | (SEQ ID NO:) | 352 nm |
| VGNYMAHMGPITWVCRPGG | (SEQ ID NO:) | 67 μM |

In addition to the foregoing, other mutagenesis studies were carried on this high affinity EPO agonist family of peptides under conditions designed to enrich for higher affinity peptides. As previously described, to enrich for higher affinity peptides, libraries are screened using an affinity selection protocol wherein the peptides are competed with EPO (100 nM). This process is typically repeated for two rounds of panning. In subsequent rounds of panning, the competition temperature (4°C to ambient temperature) and time (15 to 30 minutes) as well as the temperature of the wash solutions (4°C to ambient temperature) can be altered to further select for peptides with high affinity. Using these techniques, a mutagenesis library having three random amino acid residues on either side of the flanking sequence YXCRIGPITWVC was also screened against immobilized EPO-R (*see,* Figure 3). The mutagenesis fusion proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. Preferred peptides resulting from this study are set forth in Table 6, *infra*.

**Table 6**

GGEYICVMGPNTWVCSPTRGHGS

GGEYLCRMGPMTWVSPFTRKGG

GGQYICRFGPITWQSQPAGGGS
GREYSCRMGPITWVCMPRASLGS
GDYLCSMGPITWICVPERGGGS
ELWYSCRMGPVTWMCGRYQGGGS

In another mutagenesis study, a mutagenesis library having the strongly conserved residues (*i.e.*, Y, C, G, P, T and W) fixed, the other residues randomized and 10 additional residues at the N-terminus before the tyrosine was also screened against immobilized EPO-R *(see,* Figure 4A). The mutagenesis fusion proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. Preferred peptides resulting from this study are set forth in Table 7, *infra*.

**Table 7**

QICRADRKGIYQCWYGPETWICgg
QQGYSLWLPWYNCVLGPYTWVCgg
YGGSAAVPWKYGCSLGPVTWVCgg
QIVSWGLYSGYLCMVGPVTWLCgg
GSGALSAAGWYGCRVGPLTWVCgg
SVVSHDAAGVYDCVIGPVTWICgg
IYSWTGILGSYVCWYGPDTWVCgg
SCIYVRFFYCYQCSEGPATWLCgg
TVAKGQSGVRYSCLRGPETWVCgg
VQPQYKWATMYQCWKGPSTWFCgg
KSGVWEMGSSYQCARGPRTWCCgg
CSVRRMDREYYRCCRGPFTWQCgg
KYQEEMFMGYQCLQGPKTQLCgg
VCPGSEFRVGYICAMGPYTWDCgg
SLCSSRCNSPYFCSIGPSTWRCgg
QASLGLPLKQYLCVLGPHTWLCgg
ACKPAALFVQYGCVLGPMTWICgg
SCERAGGRWEYVCQWGPDTWLCgg
RVARQVQQVSYWCAHGPATCYCgg
HKYDTLMLTNYVCQRGPLTQLCgg

In still another mutagenesis study, a mutagenesis library having the strongly conserved residues (*i.e.*, Y, C, G, P, T and W) fixed, the other residues randomized and 10 additional residues between the second cysteine and the (Gly)4-Ser linker was also screened against immobilized EPO-R (*see,* Figure 4B). In this library, the conserved tyrosine residue was preceded by two glycine residue to allow for efficient signal peptide cleavage. The mutagenesis fusion

proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. Preferred peptides resulting from this study are set forth in Table 8, *infra*.

**Table 8**

ggYHCEWGPETWICRPEISPLTVMgg
ggYICDYGPLTWACKPAGATLLQPgg
ggYTCRFGPVTWDCLPAINHNGVLgg
ggYQ*FMGPETWVCAPEPRVERVSgg
ggYLCRFGPETWTCAPERSVVTQSgg
ggYVCDFGPTTWICRGQVMEHINTgg
ggYMCNMGPLTWDCSPVRSTSMAWgg
ggYHCEWGPETWICRPEISPLTVMgg
ggYNCTMGPNTWVCTPAAESPAVFgg
ggYGCRIGPITWICDDVSRSPRA
ggYTCRMGPQTWECLPMSEGV
ggYNCKFGPQTWDCSSANLKEV
gYLCEMGPETWMCRPEDAKLGV
ggYGCKFGPVTWICEDLLLDPMY
ggYNCKFGPQTWDCSSANLKEVLV
gYLCEMGPETWMCRPEDCEAW
ggYGCGLAPVTWECPQVSIPYGLSgg
ggYGCRIGPTTWICDSTVPQLREVgg
ggYRCSWAPETWVCDNHSA
gYLCNFGPITWDCVSSAQSEMQIgg

In yet another mutagenesis study, a mutagenesis library having the strongly conserved residues (*i.e.*, Y, C, G, P, T and W) fixed with the tyrosine placed directly next to the first cysteine and the other amino acids randomized, including 4 residues N-terminal to the tyrosine and 5 between the second cysteine and the linker, was also screened against immobilized EPO-R (*see,* Figure 4C). The N-terminal random amino acids were again preceded by two glycine residues to allow for efficient processing. The mutagenesis fusion proteins, along with the DNA encoding them, again were "panned" on immobilized EPO-R. The fusion proteins and accompanying DNA were then isolated and cultured to produce fusion protein preparations for an ELISA to determine if the fusion protein bound specifically to the receptor. Preferred peptides resulting from this study are set forth in Table 9, *infra*.

## Table 9

| |
|---|
| ggAELQYCKIGPETWVCDWPHIgg |
| ggPYEGYCSGGPVTWECCVSVCgg |
| ggQPLPYCSPGPTTWFCINWLFgg |
| ggCSYGYCPMGPFTWMCRQRRLgg |
| ggVRGSYCQSGPPTWQCDLRFFgg |
| ggRCARYCACGPGTWNCLGRCQgg |
| ggLGRCYCVYGPLTWWCSQTSLgg |
| ggLCVWYCSAGPWTWYCIYRSAgg |
| ggKPGPYCSFGPETWVCTALGMgg |
| ggRLGEYCEIGPITWICRLFLPgg |
| ggPGLGYCDFGPLTWVCDGSVDgg |
| ggLSSAYCRYGPETWICWAGTGgg |
| ggVLHLYCYYGPETWDCLPIKAgg |
| ggGGGVYCLVGPVTWLCGPAAMgg |
| ggLTRNYCRIGPETWICQEVAIgg |
| ggWSERYCVLGPLTWECVHLFAgg |
| ggMPLKYCGMGPVTWVCCEAVSgg |
| ggSVMRYCHFGPETWICPYDMPgg |
| ggALYPYCLIGPMTWVCQVGWIgg |
| ggTYGNYCRGGPGTWHCEDTRGgg |
| ggASYCYCSKGPATWKCVGSILgg |
| ggSLAAYCLQGPKTWPCVRRRLgg |
| ggTDSLYCKLGPLTWHCQLYQKgg |
| gISQQYCWRGPATWVCLEWELgg |

In addition to the foregoing mutagenesis studies, mutagenesis of peptide GGTYSCHFGPLTWVCKPQGG was performed using a modified C-terminal Lac-I display system in which display valency was reduced with the aim of identifying higher affinity hits ("Headpiece Dimer"). The Lac-I Headpiece Dimer (HPD) Display system is described in greater detail in U.S. Patent No. 5,338,665, which is hereby incorporated by reference for all purposes. Essentially, the residues which were highly conserved in the previous mutagenesis studies (*i.e.*, Y, C, G, P, T and W) were mutated lightly, whereas the less conserved residues (*i.e.*, H, F, L and V) were subject to a higher level of mutation. Four random residues proceeded the tyrosine residue, the latter being also followed by one random residue. The C-terminus of the mutagenesis scheme ended with 6 random amino acids, following the cysteine. The details of the library construction are set forth in Figure 5.

The library was screened through four rounds on PIG-tailed EPO-R immunobilized on mAb179, employing EPO elution from round two onward to enrich for higher affinity clones. The resulting DNA inserts were then cloned as a pool into a maltose binding protein (MBP) vector allowing their expression as a C-terminal fusion protein. Crude cell lysates from randomly picked individual MBP fusion clones were then assayed for EPO-R binding in an ELISA format. Preferred peptides resulting from this study are set forth in Table 10, *infra*.

**Table 10**

RTKEYSCQMGPLTWICVPKS
SKARYMCHMGPLTWVCRPEV
GGKAYMCRLGPVTWVCSPRIKL
LLRGYECYMGPLTWVCRSSKPR
TIAQYICYMGPETWECRPSPKA
NGRTYSCQLGPVTWVCSRGVRR
LGRKYSCHFGPLTWVCQPAKKD
MKTKYKCYMGPLTWVCEGS
SKTKYRCEMGPLTWVCERW
LTRLYSCHMGPSTWVCSTALRK
RGQLYACHFGPVTWVCKRRKRV
SGILYECHMGPLTWVCTPSRRR
GSKTYSCQLGPVTWVCGRKR
ARGKYQCQFGPLTWECLPIRPR

VTRMYRCRMGPLTWVCER
KPSLYECHLGPLTWECRPRRRE
RGHMYSCQLGPVTWVCKPLSGR
ITPTYHCKFGPQTWVCAPKRSALTK
GNRMYQCHMGPLTWVCQPTRIH
MKTKYKCYMGPLTWVCEGSRLK
HLGKYDCSFGPQTWVCKPRRSL
ERRVYECQMGPLTWECKPGVKG
ITPTYHCKFGPQTWVCAPKRSALTK
LGRKYSCHFGPVTWVCQPAKKD
RGRGYSCQMGPVTWVCKRERYF
RLREYRCHMGPQTWVCNGHHSK
SGALYDCQMGPITWVCRANRQK
TNQVYGCKFGPKTWVCKPAPRI
TRGMYACHMGPQTWVCRPTQPR
VLSNYECTMGPKTWVCKPLRLK

One of the most striking features of the peptides obtained using the Lac-I Headpiece Dimer Display system is the accumulation of multiple positive charges in the regions flanking the conserved cysteines, particularly so in RGQLYACHFGPVTWVCKRRKRV, which has a C-terminal stretch of 5 such residues. Amino acids which were lightly mutagenized (*i.e.*, Y, C, G, P, T and W) were absolutely conserved, whereas new motifs were generated at those positions which were more heavily mutated. Of particular interest is the presence of an additional tyrosine residue in a number of the peptides (*see, e.g.*, LLRGYECYMGPLTWVCRSSKPR), and the presence of two glutamic acid residues

between the cysteines in TTAQYICYMOPETWECRPSPKA.

In addition to the foregoing mutagenesis studies, mutagenesis of peptide TIAQYICYMGPETWECRPSPKA was performed using a modified C-terminal Lac-I display system similar to the system previously described. Essentially, the residues which were highly conserved in previous mutagenesis studies (*i.e.*, Y, C, G, P, T and W) were fixed, whereas the less conserved residues (*i.e.*, H, F, L and V) were randomized. In addition, the two glutamic acid residues were lightly mutated. Four random residues proceeded the tyrosine residue, the latter being also followed by one random residue. The C-terminus of the mutagenesis scheme ended with 6 random amino acids following the cysteine. The details of the library construction are set forth in Figure 6.

The library was screened through three rounds on PIG-tailed EPO-R immunobilized on mAb179, employing EPO elution from round two onward to enrich for higher affinity clones. Colony lifts were performed probing with the human Fcγ-EBP reagent, followed by goat anti-human Fcγ conjugated to akaline phosphatase the latter of which is available from Sigma Chemical Co., St. Louis, Missouri. Thereafter, the HPD plasmids identified were sequenced. Peptides resulting from this study are set forth in Table 11, *infra*.

**Table 11**

| |
|---|
| ALKKYDCYFGPETWECLARRPH |
| ERRFYKCRFGPETWECTL |
| FGQEYRCHLGPETWQCSPVRVG |
| FRPEYMCRMGPETWECGGARP |
| GSRKYwCRMGPETWECMKPVRL |
| GLKAYGCRYGPETWDCRSVILI |
| IRQPYICHMGPETWECGRYPAG |
| KGASYHCIMGPETWECIPQRVW |
| MKQLYSCIMGPETWECRPGVER |
| QRHYYRCALGPETWECRPMSPE |
| TKRLYHCHMGPETWECHGPMRK |
| TRPSYRCAFGPVTWECIPAR |
| RHKSYvCTFGPETWECTGAIRR |
| RGRMYNCRMGPETWECKGQSKD |
| RRRYYRCWMGPETWECSPVSNK |
| VADNYDCPIGPVTWECIHVRAS |
| VQKKYLCHFGPETWECGPDRD |
| WQTWYICERGPETWECRWLVL |
| YRMPYRCKMGPETWECVGGRGR |
| YSREYSCRMGPETWECXRGFLR |

The foregoing pool of peptide sequences were then cloned into a maltose binding protein (MBP) vector allowing their expression as a C-terminal fusion protein. Crude cell lysates from randomly picked individual MBP fusion clones were then assayed for EPO-R binding in an ELISA format. Preferred peptides resulting from this study are set forth in Table 12, *infra*.

## Table 12

| |
|---|
| RSMWYRCQMGPQTWVCGPRSAS |
| SRREYICHLGPQTWVCGPGGRK |
| GSPSYHCHLGPLTWVCKPHRMR |
| MVGRYQCHMGPRTWVCKPWHG |
| GTARYQCHFGPLTWVCKPSLKG |
| ELRGYICHFGPVTWVCKPNGSR |
| LKQGYQCQLGPQTWVCRPLRMP |
| KERKYECQFGPRTWVCQPTRAN |
| VRKVYACHMGPVTWVCVPGYKG |
| SGQRYVCRMGPETWVCRSYRGL |
| ERRSYSCQMGPVTWVCGRQMGQ |
| VKNNYRCQFGPVTWVCKAFR |
| SGASYDCQMGPITWVCRANRQK |

Representative peptides which were found to bind specifically to the EPO-receptor were also tested in cell-based functional assays. One assay utilized FDCP-1, a growth factor dependent murine multi-potential primitive hematopoietic progenitor cell line *(see, e.g.,* Dexter *et al*. (1980) *J. Exp. Med. 152*:1036-1047), as the parental cell line. This cell line can proliferate, but not differentiate, when supplemented with WEHI3-conditioned media (a medium that contains IL-3, ATCC number T1B68). The parental cell line is transfected with the human or murine EPO-R as described below to produce the FDCP-1-hEPO-R or FDCP-1-mEPO-R cell line, respectively. These transfected cell lines can proliferate, but not differentiate, in the presence of human or murine EPO.

In brief, the cells are grown to half stationary density in the presence of the necessary growth factors. The cells are then washed in PBS and starved for 16-24 hours in whole media without the growth factors. After determining the viability of the cells, stock solutions (in whole media without the growth factors) are made to give about $10^5$ cells per 50 microliters. Serial dilutions of the compounds (typically the free, solution phase peptide as opposed to a phage-bound or other bound or immobilized peptide) to be tested are made in 96-well tissue culture plates for a final volume of 50 microliters per well. Cells (50 microliters) are added to each well and the cells are incubated 24-48 hours, at which point the negative controls should die or be quiescent. Cell proliferation is then measured by techniques known in the art, such as an MTT assay which correlates with $^3$H-thymidine incorporation as an indication of cell proliferation *(see,* Mosmann (1983) *J. Immunol. Methods 65*:55). Figure 7 illustrates the results of this assay for EPO and for the peptides listed in Table 5 above. Peptides which bind to the EPO receptor and exhibit activity in the FDCP-1/hEPO-R cell based bioassay are preferred compounds of the invention.

A second cell based assay utilized the TF-1 cell line. *See,* Kitamura *et al.* (1989) *Blood 73*:375-380, which is incorporated herein by reference. Representative results are shown in Figure 8. Figure 8 depicts the effects of EPO and the free peptide VGNYMCHFGPITWVCRPGGG (SEQ ID NO:) on cellular proliferation of the cell line TF-1.

Another cell based assay which further illustrates the ability of the compounds of this invention to act as EPO agonists is based on $^3$H-thymidine incorporation into spleen cells of phenylhydrazine treated mice. The results of this assay are shown in Figures 9A-9C.

Other biological assays that can be used to demonstrate the activity of the compounds of the present invention are disclosed in Greenberger *et al.* (1983) *Proc. Natl. Acad. Sci. USA 80*:2931-2935 (EPO-dependent hematopoietic progenitor cell line); Quelle and Wojchowski (1991) *J. Biol. Chem. 266*:609-614 (protein tyrosine phosphorylation in B6SUt.EP cells); Dusanter-Fourt *et al.* (1992) *J. Biol. Chem. 287*:10670-10678 (tyrosine phosphorylation of EPO-receptor in human EPO-responsive cells); Quelle *et al*. (1992) *J. Biol. Chem. 267*:17055-17060 (tyrosine phosphorylation of a cytosolic protein (pp100) in FDC-ER cells); Worthington *et al.* (1987) *Exp. Hematol. 15*:85-92 (colorimetric assay for hemoglobin); Kaiho and Miuno (1985) *Anal. Biochem. 149*:117-120 (detection of hemoglobin with 2,7-diaminofluorene); Patel *et al.* (1992) *J. Biol. Chem. 267*:21300-21302 (expression of *c-myb*; Witthuhn *et al.* (1993) *Cell 74*:227-236 (association and tyrosine phosphorylation of JAK2); Leonard *et al.* (1993) *Blood 82*:1071-1079 (expression of GATA transcription factors); Ando *et al.* (1993) *Proc. Notl. Acad. Sci. USA 90*:9571-9575 (regulation of $G_1$/3 transition by cycling D2 and D3); and calcium flux, each of which is incorporated herein by reference.

An instrument designed by Molecular Devices Corp., known as a microphysiometer, has been reported to be successfully used for measurement of the effect of agonists and antagonists on various receptors. The basis for this apparatus is the measurement of the alterations in the acidification rate of the extracellular media in response to receptor activation.

According to a preferred embodiment, the peptides of the present invention are dimerized or oligomerized, thereby increasing the affinity and/or activity of the lead compounds disclosed herein. To investigate the effect that peptide dimerization/oligomerization has on EPO mimetic potency in cell proliferation assays, a C-terminally biotinylated analog of GGTYSCHFGPLTWVCKPQGG was synthesized (*i.e.*, GGTYSCHFGPLTWVCKPQGGSSK (Ahx-biotin)).

This peptide was incubated with streptavidin in PBS at a 4:1 molar ratio at room temperature for one hour. Following this, the complex was introduced into the PIG-tailed EPO-R binding assay for $IC_{50}$ determination. Peptide that had not been complexed to streptavidin was also run in the same experiment. Pre-complexed peptide was found to have an $IC_{50}$ of 20 nM compared to the 350 nM $IC_{50}$ of peptide that had not been incubated with streptavidin. This greater than 10-fold increase in apparent affinity is presumably due to the multivalent state of the peptide-streptavidin complex. Since this comparison was made using the free peptide concentrations and not the effective complex concentration (theoretically 4-fold lower), the effect will be even greater.

In addition, this peptide was preincubated with streptavidin in serum-free HEPES-buffered RPMI at a 4:1 molar ratio, as above. The complex was tested for stimulation of cell proliferation in an FDCP-1/hEPO-R bioassay alongside free GGTYSCHFGPLTWVCKPQGGSSK (Ahx-biotin)) and the unbiotinylated parental peptide, i.e., GGTYSCHFGPLTWVCKPQGG. Figure 10 illustrates the results of the assay where the EPO-$ED_{50}$ of the free peptides were similar (approximately 1 μM), but that of the pre-formed streptavidin complex was less than 10 nM, a two log reduction in EPO-$ED_{50}$. Streptavidin, alone, had some small stimulatory effect at the highest concentrations, presumably due to contamination with bacterial endotoxin.

In addition, an increase in biological potency was found when the peptide was dimerization with goat anti-biotin IgG. Figure 11 illustrates that a one log reduction in EPO-$ED_{50}$ can also be achieved by preincubation of GGTYSCHFGPLTWVCKPQGGSSK (Ahx-biotin) with purified goat anti-biotin IgG at a 2:1 molar ratio. This increase in biological potency is presumably due to the dimerization of the peptide by the antibodies. The anti-biotin antibodies alone had no effect on the cells. Moreover, a 100-fold reduction in EPO-$ED_{50}$ was seen with the GGTYSCHFGPLTWVCKPQGGSSK (Ahx-biotin)-streptavidin complex. As such, by dimerizing or oligomerizing the lead peptides of the present invention, the affinity and/or activity of such peptides can be increased.

In another embodiment, a dimeric peptide analog of GGTYSCHFGPLTWVCKPQGG containing two disulfide bonds was prepared using the general scheme set forth in Figure 12. Briefly, the first peptide chain was assembled on a Tentagel resin. The Fmoc-Lys(Alloc) was coupled to the Knorr linker, the Alloc group being used as an orthogonal protecting group. For the first peptide chain, Cys(Acm) was used. After the completion of the first peptide chain, the Alloc group was removed and the second peptide chain was built upon the side chain amines of the lysine residue. In this peptide chain, Cys(trt) was used. After the synthesis was completed, the peptide was cleaved from the resin and purified. The peptide was then cyclized to a compound containing a single disulfide bond. Thereafter, the second disulfide bond was formed by iodine oxidation, yielding the bicyclic dimer.

Figures 13 and 14 show the *in vitro* EPOR binding and biological activities of the dimer. The affinity of the dimer was tested against the PIG-tailed EPOR immobilized on mAb179 in strip wells. The result of the equilibrium competition binding assay indicated an affinity of approximately 2 nM, a 200-fold increase over the value for the parental peptide, GGTYSCHFGPLTWVCKPQGG, (200 nM), and 5-fold over the GGTYSCHFGPLTWVCKPQGGSSK (Alu-biotin)-streptavidin complex. *In vitro* bioactivity, as measured by FDCP-1/hEPOR cell proliferation, was increased approximately 20-fold from an $EC_{50}$ of 400 nM (for the parental peptide) to 20 nM. As such, by dimerizing or oligomerizing the lead peptides of the present invention, the affinity and/or activity of such peptides can be significantly increased.

The peptides of this invention or derivatives thereof can be conjugated to compounds that bind to the EPO-R to construct compounds with an affinity for the receptor greater than either of the compounds of which the conjugate is composed. The discovery of these peptides also facilitates the identification of peptides that bind to the same site on the EPO-R, because one can bias the library or panning procedure to eliminate peptides with the complementary "non-blocking" motif.

The preferred motif sequence also provides a means to determine the minimum size of an EPO agonist of the invention. Using the "encoded synthetic library" (ESL) system described in U.S. patent application Serial No. 946,239, filed September 16, 1992, which is a continuation-in-part application of Serial No. 762,522, filed September 18, 1991, or the "very large scale immobilized polymer synthesis" system described in U.S. patent application Serial Nos. 492,462, filed March 7, 1990; 624,120, filed December 6, 1990; and 805,727, filed December 6, 1991; one can not only determine the minimum size of a peptide with such activity, one can also make all of the peptides that form the group of peptides that differ from the preferred motif (or the minimum size of that motif) in one, two, or more residues. This collection of peptides can then be screened for the ability to bind to EPO-receptor. This immobilized polymer synthesis system or other peptide synthesis methods can also be used to synthesize every truncation analog and every

deletion analog and every combination of truncation and deletion analog of all of the peptide compounds of the invention.

The peptides of the invention can also be prepared by classical methods known in the art, for example, by using standard solid phase techniques. The standard methods include exclusive solid phase synthesis, partial solid phase synthesis methods, fragment condensation, classical solution synthesis, and even by recombinant DNA technology. *See, e.g.,* Merrifield, 1963, *J. Am. Chem. Soc. 85*:2149, incorporated herein by reference. On solid phase, the synthesis is typically commenced from the C-terminal end of the peptide using an α-amino protected resin. A suitable starting material can be prepared, for instance, by attaching the required α-amino acid to a chloromethylated resin, a hydroxymethyl resin, or a benzhydrylamine resin. One such chloromethylated resin is sold under the tradename BIO-BEADS SX-1 by Bio Rad Laboratories, Richmond, CA, and the preparation of the hydroxymethyl resin is described by Bodonszky *et al*., 1966, *Chem. Ind*. (London) *38*:1597. The benzhydrylamine (BHA) resin has been described by Pietta and Marshall, 1970, *Chem. Commn*. 650, and is commercially available from Beckman Instruments, Inc., Palo Alto, CA, in the hydrochloride form.

Thus, the compounds of the invention can be prepared by coupling an α-amino protected amino acid to the chloromethylated resin with the aid of, for example, cesium bicarbonate catalyst, according to the method described by Gisin, 1973, *Helv. Chim. Acta 56*:1467. After the initial coupling, the α-amino protecting group is removed by a choice of reagents including trifluoroacetic acid (TFA) or hydrochloric acid (HCl) solutions in organic solvents at room temperature.

The α-amino protecting groups are those known to be useful in the art of stepwise synthesis of peptides. Included are acyl type protecting groups (*e.g.*, formyl, trifluoroacetyl, acetyl), aromatic urethane type protecting groups (*e.g.*, benzyloxycarboyl (Cbz) and substituted Cbz), aliphatic urethane protecting groups (*e.g.*, t-butyloxycarbonyl (Boc), isopropyloxycarbonyl, cyclohexyloxycarbonyl) and alkyl type protecting groups (*e.g.*, benzyl, triphenylmethyl). Fmoc is a preferred protecting group. The side chain protecting group (typically ethers, esters, trityl, PMC, and the like) remains intact during coupling and is not split off during the deprotection of the amino-terminus protecting group or during coupling. The side chain protecting group must be removable upon the completion of the synthesis of the final peptide and under reaction conditions that will not alter the target peptide.

The side chain protecting groups for Tyr include tetrahydropyranyl, tert-butyl, trityl, benzyl, Cbz, Z-Br-Cbz, and 2,5-dichlorobenzyl. The side chain protecting groups for Asp include benzyl, 2,6-dichlorobenzyl, methyl, ethyl, and cyclohexyl. The side chain protecting groups for Thr and Ser include acetyl, benzoyl, trityl, tetrahydropyranyl, benzyl, 2,6-dichlorobenzyl, and Cbz. The side chain protecting groups for Thr and Ser are benzyl. The side chain protecting groups for Arg include nitro, Tosyl (Tos), Cbz, adamantyloxycarbonyl mesitoylsulfonyl (Mts), or Boc. The side chain protecting groups for Lys include Cbz, 2-chlorobenzyloxycarbonyl (2-Cl-Cbz), 2-bromobenzyloxycarbonyl (2-BrCbz), Tos, or Boc.

After removal of the α-amino protecting group, the remaining protected amino acids are coupled stepwise in the desired order. Each protected amino acid is generally reacted in about a 3-fold excess using an appropriate carboxyl group activator such as 2-(1H-benzotriazol-1-yl)-1,1,3,3tetramethyluronium hexafluorophosphate (HBTU) or dicyclohexylcarbodiimide (DCC) in solution, for example, in methylene chloride ($CH_2Cl_2$), dimethyl formamide (DMF) mixtures.

After the desired amino acid sequence has been completed, the desired peptide is decoupled from the resin support by treatment with a reagent such as trifluoroacetic acid (TFA) or hydrogen fluoride (HF), which not only cleaves the peptide from the resin, but also cleaves all remaining side chain protecting groups. When the chloromethylated resin is used, hydrogen fluoride treatment results in the formation of the free peptide acids. When the benzhydrylamine resin is used, hydrogen fluoride treatment results directly in the free peptide amide. Alternatively, when the chloromethylated resin is employed, the side chain protected peptide can be decoupled by treatment of the peptide resin with ammonia to give the desired side chain protected amide or with an alkylamine to give a side chain protected alkylamide or dialkylamide. Side chain protection is then removed in the usual fashion by treatment with hydrogen fluoride to give the free amides, alkylamides, or dialkylamides.

In preparing the esters of the invention, the resins used to prepare the peptide acids are employed, and the side chain protected peptide is cleaved with base and the appropriate alcohol, *i.e.*, methanol. Side chain protecting groups are then removed in the usual fashion by treatment with hydrogen fluoride to obtain the desired ester. These solid phase peptide synthesis procedures are well-known in the art and further described in Stewart, *Solid Phase Peptide Syntheses* (Freeman and Co., San Francisco, 1969).

These procedures can also be used to synthesize peptides in which amino acids other than the 20 naturally occurring, genetically encoded amino acids are substituted at one, two, or more positions of any of the compounds of the invention. For instance, naphthylalanine can be substituted for tryptophan, facilitating synthesis. Other synthetic amino acids that can be substituted into the peptides of the present invention include L-hydroxypropyl, L-3, 4-dihydroxyphenylalanyl, δ amino acids such as L-δ-hydroxylysyl and D-δ-methylalanyl, L-α-methylalanyl, β amino acids, and isoquinolyl. D-amino acids and non-naturally occurring synthetic amino acids can also be incorporated into the

peptides of the present invention.

One can replace the naturally occurring side chains of the 20 genetically encoded amino acids (or D amino acids) with other side chains, for instance with groups such as alkyl, lower alkyl, cyclic 4-, 5-, 6-, to 7-membered alkyl, amide, amide lower alkyl, amide di(lower alkyl), lower alkoxy, hydroxy, carboxy and the lower ester derivatives thereof, and with 4-, 5-, 6-, to 7-membered hetereocyclic. In particular, proline analogs in which the ring size of the proline residue is changed from 5 members to 4, 6, or 7 members can be employed. Cyclic groups can be saturated or unsaturated, and if unsaturated, can be aromatic or non-aromatic.

Cyclic groups can be saturated or unsaturated, and if unsaturated, can be aromatic or non-aromatic. Heterocyclic groups preferably contain one or more nitrogen, oxygen, and/or sulphur heteroatoms. Examples of such groups include the furazanyl, furyl, imidazolidinyl, imidazolyl, imidazolinyl, isothiazolyl, isoxazolyl, morpholinyl (e.g. morpholino), oxazolyl, piperazinyl (e.g. 1-piperazinyl), piperidyl (e.g. 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g. 1-pyrrolidinyl), pyrrolinyl, pyrrolyl, thiadiazolyl, thiazolyl, thienyl, thiomorpholinyl (e.g. thiomorpholino), and triazolyl. These heterocyclic groups can be substituted or unsubstituted. Where a group is substituted, the substituent can be alkyl, alkoxy, halogen, oxygen, or substituted or unsubstituted phenyl.

One can also readily modify the peptides of the instant invention by phosphorylation, and other methods for making peptide derivatives of the compounds of the present invention are described in Hruby et al.[42] Thus, the peptide compounds of the invention also serve as a basis to prepare peptide mimetics with similar biological activity.

The peptide compounds of the invention, including peptidomimetics, can be covalently modified to one or more of a variety of nonproteinaceous polymers, *e.g.*, polyethylene glycol, polypropylene glycol, or polyoxyalkenes, in the manner set forth in U.S. Patent No. 4,640,835; U.S. Patent No. 4,496,689; U.S. Patent No. 4,301,144; U.S. Patent No. 4,670,417; U.S. Patent No. 4,791,192; or U.S. Patent No. 4,179,337, all which are incorporated by reference in their entirety herein.

Those of skill in the art recognize that a variety of techniques are available for constructing peptide mimetics with the same or similar desired biological activity as the corresponding peptide compound but with more favorable activity than the peptide with respect to solubility, stability, and susceptibility to hydrolysis and proteolysis. *See,* for example, Morgan and Gainor *Ann. Rep. Med. Chem.* **24:243-252** (1989). The following describes methods for preparing peptide mimetics modified at the N-terminal amino group, the C-terminal carboxyl group, and/or changing one or more of the amido linkages in the peptide to a non-amido linkage. It being understood that two or more such modifications can be coupled in one peptide mimetic structure (*e.g.*, modification at the C-terminal carboxyl group and inclusion of a $-CH_2$-carbamate linkage between two amino acids in the peptide).

The peptides typically are synthesized as the free acid but, as noted above, could be readily prepared as the amide or ester. One can also modify the amino and/or carboxy terminus of the peptide compounds of the invention to produce other compounds of the invention. Amino terminus modifications include methylating (*i.e.*, $-NHCH_3$ or $-NH(CH_3)_2$), acetylating, adding a carbobenzoyl group, or blocking the amino terminus with any blocking group containing a carboxylate functionality defined by RCOO-, where R is selected from the group consisting of naphthyl, acridinyl, steroidyl, and similar groups. Carboxy terminus modifications include replacing the free acid with a carboxamide group or forming a cyclic lactam at the carboxy terminus to introduce structural constraints.

Amino terminus modifications are as recited above and include alkylating, acetylating, adding a carbobenzoyl group, forming a succinimide group, etc. Specifically, the N-terminal amino group can then be reacted as follows:

(a) to form an amide group of the formula RC(O)NH- where R is as defined above by reaction with an acid halide [*e.g.*, RC(O)Cl] or acid anhydride. Typically, the reaction can be conducted by contacting about equimolar or excess amounts (*e.g.*, about 5 equivalents) of an acid halide to the peptide in an inert diluent (*e.g.*, dichloromethane) preferably containing an excess (*e.g.*, about 10 equivalents) of a tertiary amine, such as diisopropylethylamine, to scavenge the acid generated during reaction. Reaction conditions are otherwise conventional (*e.g.*, room temperature for 30 minutes). Alkylation of the terminal amino to provide for a lower alkyl N-substitution followed by reaction with an acid halide as described above will provide for N-alkyl amide group of the formula RC(O)NR-;

(b) to form a succinimide group by reaction with succinic anhydride. As before, an approximately equimolar amount or an excess of succinic anhydride (*e.g.*, about 5 equivalents) can be employed and the amino group is converted to the succinimide by methods well known in the art including the use of an excess (*e.g.*, ten equivalents) of a tertiary amine such as diisopropylethylamine in a suitable inert solvent (*e.g.*, dichloromethane). *See,* for example, Wollenberg, et al., U.S. Patent No. 4,612,132 which is incorporated herein by reference in its entirety. It is understood that the succinic group can be substituted with, for example, $C_2$-$C_6$ alkyl or -SR substituents which are prepared in a conventional manner to provide for substituted succinimide at the N-terminus of the peptide. Such alkyl substituents are prepared by reaction of a lower olefin ($C_2$-$C_6$) with maleic anhydride in the manner described by Wollenberg, et al., supra. and -SR substituents are prepared by reaction of RSH with maleic anhydride where R is as defined above;

(c) to form a benzyloxycarbonyl-NH- or a substituted benzyloxycarbonyl-NH- group by reaction with approximately an equivalent amount or an excess of CBZ-Cl (*i.e.*, benzyloxycarbonyl chloride) or a substituted CBZ-Cl in a suitable inert diluent (*e.g.*, dichloromethane) preferably containing a tertiary amine to scavenge the acid generated during the reaction;

(d) to form a sulfonamide group by reaction with an equivalent amount or an excess (*e.g.*, 5 equivalents) of R-S $(O)_2Cl$ in a suitable inert diluent (dichloromethane) to convert the terminal amine into a sulfonamide where R is as defined above. Preferably, the inert diluent contains excess tertiary amine (e.g., ten equivalents) such as diisopropylethylamine, to scavenge the acid generated during reaction. Reaction conditions are otherwise conventional (e.g., room temperature for 30 minutes);

(e) to form a carbamate group by reaction with an equivalent amount or an excess (*e.g.*, 5 equivalents) of R-OC (O)Cl or R-OC(O)$OC_6H_4$-p-$NO_2$ in a suitable inert diluent (*e.g.*, dichloromethane) to convert the terminal amine into a carbamate where R is as defined above. Preferably, the inert diluent contains an excess (*e.g.*, about 10 equivalents) of a tertiary amine, such as diisopropylethylamine, to scavenge any acid generated during reaction. Reaction conditions are otherwise conventional (*e.g.*, room temperature for 30 minutes); and

(f) to form a urea group by reaction with an equivalent amount or an excess (*e.g.*, 5 equivalents) of R-N=C=O in a suitable inert diluent (*e.g.*, dichloromethane) to convert the terminal amine into a urea (*i.e.*, RNHC(O)NH-) group where R is as defined above. Preferably, the inert diluent contains an excess (*e.g.*, about 10 equivalents) of a tertiary amine, such as diisopropylethylamine. Reaction conditions are otherwise conventional (*e.g.*, room temperature for about 30 minutes).

Additionally, or alternatively, the C-terminus can be modified. In preparing peptide mimetics wherein the C-terminal carboxyl group is replaced by an ester (i.e., -C(O)OR where R is as defined above), the resins used to prepare the peptide acids are employed, and the side chain protected peptide is cleaved with base and the appropriate alcohol, *e.g.*, methanol. Side chain protecting groups are then removed in the usual fashion by treatment with hydrogen fluoride to obtain the desired ester.

In preparing peptide mimetics wherein the C-terminal carboxyl group is replaced by the amide -C(O)$NR^3R^4$, a benzhydrylamine resin is used as the solid support for peptide synthesis. Upon completion of the synthesis, hydrogen fluoride treatment to release the peptide from the support results directly in the free peptide amide (*i.e.*, the C-terminus is -C(O)$NH_2$). Alternatively, use of the chloromethylated resin during peptide synthesis coupled with reaction with ammonia to cleave the side chain protected peptide from the support yields the free peptide amide and reaction with an alkylamine or a dialkylamine yields a side chain protected alkylamide or dialkylamide (*i.e.*, the C-terminus is -C(O) $NRR^1$ where R and $R^1$ are as defined above). Side chain protection is then removed in the usual fashion by treatment with hydrogen fluoride to give the free amides, alkylamides, or dialkylamides.

In another alternative embodiment, the C-terminal carboxyl group or a C-terminal ester can be induced to cyclize by internal displacement of the -OH or the ester (-OR) of the carboxyl group or ester respectively with the N-terminal amino group to form a cyclic peptide. For example, after synthesis and cleavage to give the peptide acid, the free acid is converted to an activated ester by an appropriate carboxyl group activator such as dicyclohexylcarbodiimide (DCC) in solution, for example, in methylene chloride ($CH_2Cl_2$), dimethyl formamide (DMF) mixtures. The cyclic peptide is then formed by internal displacement of the activated ester with the N-terminal amine. Internal cyclization as opposed to polymerization can be enhanced by use of very dilute solutions. Such methods are well known in the art.

One can also cyclize the peptides of the invention, or incorporate a desamino or descarboxy residue at the terminii of the peptide, so that there is no terminal amino or carboxyl group, to decrease susceptibility to proteases or to restrict the conformation of the peptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

Other methods for making peptide derivatives of the compounds of the present invention are described in Hruby, *et al*., *Biochem J.* **268(2):249-262** (1990), incorporated herein by reference. Thus, the peptide compounds of the invention also serve as structural models for non-peptidic compounds with similar biological activity. Those of skill in the art recognize that a variety of techniques are available for constructing compounds with the same or similar desired biological activity as the lead peptide compound but with more favorable activity than the lead with respect to solubility, stability, and susceptibility to hydrolysis and proteolysis. *See*, Morgan and Gainor, *Ann. Rep. Med. Chem.* **24:243-252** (1989), incorporated herein by reference. These techniques include replacing the peptide backbone with a backbone composed of phosphonates, amidates, carbamates, sulfonamides, secondary amines, and N-methylamino acids.

Peptide mimetics wherein one or more of the peptidyl linkages [-C(O)NH-] have been replaced by such linkages as a -$CH_2$-carbamate linkage, a phosphonate linkage, a -$CH_2$-sulfonamide linkage, a urea linkage, a secondary amine (-$CH_2$NH-) linkage, and an alkylated peptidyl linkage [-C(O)$NR^6$- where $R^6$ is lower alkyl] are prepared during conventional peptide synthesis by merely substituting a suitably protected amino acid analogue for the amino acid

reagent at the appropriate point during synthesis.

Suitable reagents include, for example, amino acid analogues wherein the carboxyl group of the amino acid has been replaced with a moiety suitable for forming one of the above linkages. For example, if one desires to replace a -C(O)NR- linkage in the peptide with a $-CH_2$-carbamate linkage ($-CH_2OC(O)NR-$), then the carboxyl (-COOH) group of a suitably protected amino acid is first reduced to the $-CH_2OH$ group which is then converted by conventional methods to a -OC(O)Cl functionality or a para-nitrocarbonate $-OC(O)O-C_6H_4$-p-$NO_2$ functionality. Reaction of either of such functional groups with the free amine or an alkylated amine on the N-terminus of the partially fabricated peptide found on the solid support leads to the formation of a $-CH_2OC(O)NR-$ linkage. For a more detailed description of the formation of such $-CH_2$-carbamate linkages, *see,* Cho, *et al. Science,* **261:1303-1305** (1993).

Similarly, replacement of an amido linkage in the peptide with a phosphonate linkage can be achieved in the manner set forth in U.S. Patent Application Serial Nos. 07/943,805, 08/081,577, and 08/119,700, the disclosures of which are incorporated herein by reference in their entirety.

Replacement of an amido linkage in the peptide with a - $CH_2$-sulfonamide linkage can be achieved by reducing the carboxyl (-COOH) group of a suitably protected amino acid to the $-CH_2OH$ group and the hydroxyl group is then converted to a suitable leaving group such as a tosyl group by conventional methods. Reaction of the tosylated derivative with, for example, thioacetic acid followed by hydrolysis and oxidative chlorination will provide for the $-CH_2-S(O)_2Cl$ functional group which replaces the carboxyl group of the otherwise suitably protected amino acid. Use of this suitably protected amino acid analogue in peptide synthesis provides for inclusion of an $-CH_2S(O)_2NR-$ linkage which replaces the amido linkage in the peptide thereby providing a peptide mimetic. For a more complete description on the conversion of the carboxyl group of the amino acid to a $-CH_2S(O)_2Cl$ group, *see,* for example, Weinstein, Boris, *Chemistry & Biochemistry of Amino Acids, Peptides and Proteins*, vol. 7, pp. 267-357, Marcel Dekker, Inc., New York (1983) which is incorporated herein by reference.

Replacement of an amido linkage in the peptide with a urea linkage can be achieved in the manner set forth in U.S. Patent Application Serial No. 08/147,805 which application is incorporated herein by reference in its entirety.

Secondary amine linkages wherein a $-CH_2NH-$ linkage replaces the amido linkage in the peptide can be prepared by employing, for example, a suitably protected dipeptide analogue wherein the carbonyl bond of the amido linkage has been reduced to a $CH_2$ group by conventional methods. For example, in the case of diglycine, reduction of the amide to the amine will yield after deprotection $H_2NCH_2CH_2NHCH_2COOH$ which is then used in N-protected form in the next coupling reaction. The preparation of such analogues by reduction of the carbonyl group of the amido linkage in the dipeptide is well known in the art.

The suitably protected amino acid analogue is employed in the conventional peptide synthesis in the same manner as would the corresponding amino acid. For example, typically about 3 equivalents of the protected amino acid analogue are employed in this reaction. An inert organic diluent such as methylene chloride or DMF is employed and, when an acid is generated as a reaction by-product, the reaction solvent will typically contain an excess amount of a tertiary amine to scavenge the acid generated during the reaction. One particularly preferred tertiary amine is diisopropylethylamine which is typically employed in about 10 fold excess. The reaction results in incorporation into the peptide mimetic of an amino acid analogue having a non-peptidyl linkage. Such substitution can be repeated as desired such that from zero to all of the amido bonds in the peptide have been replaced by non-amido bonds.

One can also cyclize the peptides of the invention, or incorporate a desamino or descarboxy residue at the terminii of the peptide, so that there is no terminal amino or carboxyl group, to decrease susceptibility to proteases or to restrict the conformation of the peptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

According to another preferred embodiment, residues $X_3$ and $X_8$ will be independently selected from the group of C and Hoc. Thus, the compounds of the present invention may exist in a cyclized form with an intramolecular disulfide bond. Alternatively, an intermolecular disulfide bond can be produced to yield a dimeric compound. These intramolecular or intermolecular disulfide derivatives can be represented schematically as shown below:

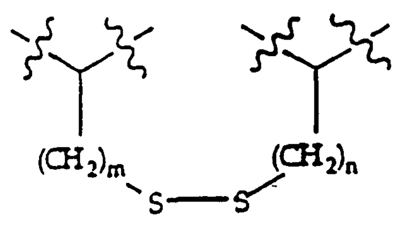

wherein m and n are independently 1 or 2.

Other embodiments of this invention provide for analogues of these disulfide derivatives in which one of the sulfurs has been replaced by a $CH_2$ group or other isostere for sulfur. These analogues can be prepared from the compounds of the present invention wherein either $X_3$ or $X_8$ is C or Hoc and the other is α-amino-γ-butyric acid, via an intramolecular or intermolecular displacement, using methods known in the art as shown below:

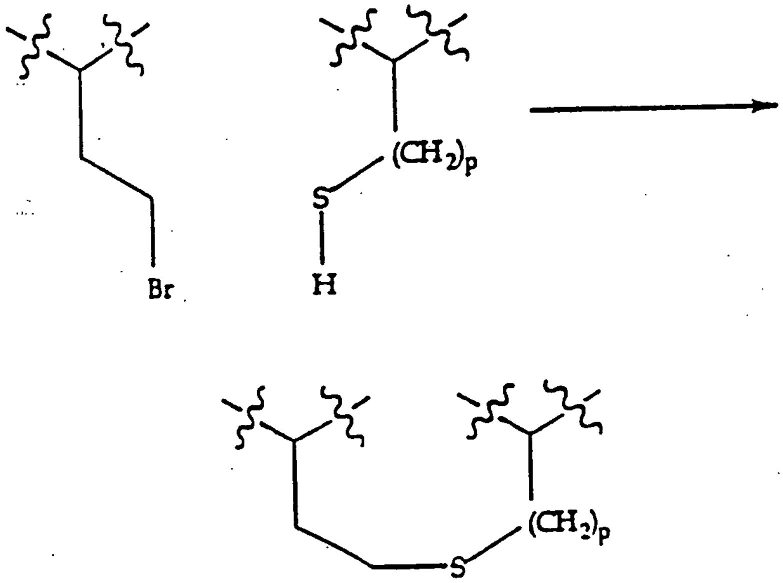

wherein p is 1 or 2. One of skill in the art will readily appreciate that this displacement can also occur using other homologs of α-amino-γ-butyric acid and homocysteine.

In addition to the foregoing cyclization strategies, other non-disulfide peptide cyclization strategies can be employed. Such alternative cyclization strategies include, for example, amide-cyclization strategies as well as cyclization strategies involving the formation of thio-ether bonds. Thus, the compounds of the present invention can exist in a cyclized form with either an intramolecular amide bond or an intramolecular thio-ether bond. To illustrate the feasibility of the amide-cyclization strategy, a peptide based on ggTYSCHFGPLTWVCKPQgg was synthesized wherein the first cysteine was replaced with lysine, the second cysteine was replaced with glutamic acid, and a cyclic monomer was formed through an amide bond between the side chains of these two residues. In addition, to illustrate the feasibility of the thio-ether cyclization strategy, a peptide based on ggTYSCHFGPLTWVCKPQgg was synthesized wherein the first cysteine was replaced with lysine and a cyclic monomer was formed through a thio-ether linkage between the side chains of the lysine residue and the C-terminal cysteine. As such, in addition to disulfide cyclization strategies, amide-cyclization strategies and thio-ether cyclization strategies can both be readily used to cyclize the compounds of the present invention.

Alternatively, the amino-terminus of the peptide can be capped with an *alpha*-substituted acetic acid, wherein the alpha substituent is a leaving group, such as an α-haloacetic acid, for example, α-chloroacetic acid, α-bromoacetic acid, or α-iodoacetic acid. The compounds of the present invention wherein $X_3$ is C or Hoc can be cyclized via displacement of the leaving group by the sulfur of the $X_3$ residue. *See, e.g.,* Barker *et al*. (1992) *J. Med. Chem. 35*: 2040-2048 and Or *et al.* (1991) *J. Org. Chem. 56*:3146-3149, each of which is incorporated herein by reference.

The compounds of the invention are useful *in vitro* as unique tools for understanding the biological role of EPO, including the evaluation of the many factors thought to influence, and be influenced by, the production of EPO and the binding of EPO to the EPO-R *(e.g.,* the mechanism of EPO signal transduction/receptor activation). The present compounds are also useful in the development of other compounds that bind to the EPO-R, because the present compounds provide important structure-activity relationship (SAR) information that facilitate that development.

Moreover, based on their ability to bind to the EPO receptor, the peptides of the present invention can be used as reagents for detecting EPO receptors on living cells, fixed cells, in biological fluids, in tissue homogenates, in purified, natural biological materials, *etc*. For example, by labelling such peptides, one can identify cells having EPO-R on their surfaces. In addition, based on their ability to bind the EPO receptor, the peptides of the present invention can be used in *in situ* staining, FACS (fluorescence-activated cell sorting), Western blotting, ELISA (enzyme-linked immunoadsoptive assay), *etc.* In addition, based on their ability to bind to the EPO receptor, the peptides of the present

invention can be used in receptor purification, or in purifying cells expressing EPO receptors on the cell surface (or inside permeabilized cells).

The compounds of the invention can also be utilized as commercial research reagents for various medical research and diagnostic uses. Such uses can include but are not limited to: (1) use as a calibration standard for quantitating the activities of candidate EPO agonists in a variety of functional assays; (2) use as blocking reagents in random peptide screening, *i.e.*, in looking for new families of EPO receptor peptide ligands, the peptides can be used to block recovery of the presently claimed EPO peptides; (3) use in the co-crystallization with EPO receptor, *i.e.*, the peptides of the present invention will allow formation of crystals bound to the EPO receptor, enabling the determination of receptor/peptide structure x-ray crystallography; (4) use to measure the capacity of erythrocyte precursor cells to differentiate and thus, stimulate the induction of globin synthesis and increases in the synthesis of the heme complex and in the number of ferritin receptors; (5) use to maintain the proliferation and growth of EPO-dependent cell lines, such as the FDCP-1-mEPO-R and the TF-1 cell lines; and (6) other research and diagnostic applications wherein the EPO-receptor is preferably activated or such activation is conveniently calibrated against a known quantity of an EPO agonist, and the like.

The compounds of the invention can also be administered to warm blooded animals, including humans, to simulate the binding of EPO to the EPO-R *in vivo*. Thus, the present invention encompasses methods for therapeutic treatment of disorders associated with a deficiency of EPO that comprise administering a compound of the invention in amounts sufficient to stimulate the EPO-R and thus, alleviate the symptoms associated with a deficiency of EPO *in vivo*. For example, the compounds of this invention will find use in the treatment of end-stage renal failure/dialysis; anemia associated with AIDS, anemia associated with chronic inflammatory diseases (for example, rheumatoid arthritis and chronic bowel inflammation), autoimmune disease, and malignancies; and for boosting the red blood count of a patient prior to surgery.

Other embodiments of this invention provide for the administration of the compounds of the invention for the treatment of disorders which are not characterized by low or deficient red blood cells, for example as a pretreatment prior to transfusions. In addition, administration of the compounds of this invention can result in a decrease in bleeding time and thus, will find use in the administration to patients prior to surgery or for indications wherein bleeding is expected to occur. In addition, the compounds of this invention will find use in the activation of megakaryoctes.

Since EPO has been shown to have a mitogenic and chemotactic effect on vascular endothelial cells as well as an effect on central cholinergic neurons (*see*, *e.g*., Amagnostou *et al*. (1990) *Proc. Natl. Acad. Sci. USA 87*: 5978-5982 and Konishi *et al*. (1993) *Brain Res. 609*:29-35), the compounds of this invention will also find use for the treatment of a variety of vascular disorders, such as promoting wound healing, growth of collateral coronary blood vessels (such as those that may occur after myocardial infarction), trauma, and post-vascular graft treatment, and a variety of neurological disorders, generally characterized by low absolute levels of acetyl choline or low relative levels of acetyl choline as compared to other neuroactive substances *e.g*., neurotransmitters.

Accordingly, the present invention includes pharmaceutical compositions comprising, as an active ingredient, at least one of the peptides or other compounds of the invention in association with a pharmaceutical carrier or diluent. The compounds of this invention can be administered by oral, parenteral (intramuscular, intraperitoneal, intravenous (IV) or subcutaneous injection), transdermal (either passively or using iontophoresis or electroporation), transmucosal (nasal, vaginal, rectal, or sublingual) routes of administration or using bioerodible inserts and can be formulated in dosage forms appropriate for each route of administration.

Solid dosage forms for oral administration include capsules, tablets, pills, powders, and granules. In such solid dosage forms, the active compound is admixed with at least one inert pharmaceutically acceptable carrier such as sucrose, lactose, or starch. Such dosage forms can also comprise, as is normal practice, additional substances other than inert diluents, e. g. , lubricating, agents such as magnesium stearate. In the case of capsules, tablets, and pills, the dosage forms may also comprise buffering, agents. Tablets and pills can additionally be prepared with enteric coatings.

Liquid dosage forms for oral administration include pharmaceutically acceptable emulsions, solutions, suspensions, syrups, with the elixirs containing inert diluents commonly used in the art, such as water. Besides such inert diluents, compositions can also include adjuvants, such as wetting agents, emulsifying and suspending agents, and sweetening, flavoring, and perfuming agents.

Preparations according to this invention for parenteral administration include sterile aqueous or non-aqueous solutions, suspensions, or emulsions. Examples of non-aqueous solvents or vehicles are propylene glycol, polyethylene glycol, vegetable oils, such as olive oil and corn oil, gelatin, and injectable organic esters such as ethyl oleate. Such dosage forms may also contain adjuvants such as preserving, wetting, emulsifying, and dispersing agents. They may be sterilized by, for example, filtration through a bacteria retaining filter, by incorporating sterilizing agents into the compositions, by irradiating the compositions, or by heating the compositions. They can also be manufactured using sterile water, or some other sterile injectable medium, immediately before use.

Compositions for rectal or vaginal administration are preferably suppositories which may contain, in addition

to the active substance, excipients such as cocoa butter or a suppository wax. Compositions for nasal or sublingual administration are also prepared with standard excipients well known in the art.

The dosage of active ingredient in the compositions of this invention may be varied; however, it is necessary that the amount of the active ingredient shall be such that a suitable dosage form is obtained. The selected dosage depends upon the desired therapeutic effect, on the route of administration, and on the duration of the treatment desired. Generally dosage levels of between 0.001 to 10 mg/kg of body weight daily are administered to mammals.

As can be appreciated from the disclosure above, the present invention has a wide variety of applications. Accordingly, the following examples are offered by way of illustration, not by way of limitation.

EXAMPLE 1

Solid Phase Peptide Synthesis

Various peptides of the invention were synthesized using the Merrifield solid phase synthesis techniques *(see,* Stewart, J.M., and Young, J.D., *Solid Phase Peptide Synthesis*, 2d. edition (Pierce Chemical, Rockford, IL, 1984)) on a Milligen/Biosearch 9600 automated instrument. The resin used was PAL (Milligen/Biosearch), which is cross-linked polystyrene with 5-(4'-Fmoc-aminomethyl-3,5'-dimethoxyphenoxy) valeric acid as a linker. Use of PAL resin results in a carboxyl terminal amide function upon cleavage of the peptide from the resin. Primary amine protection on amino acids was achieved with F-moc, and side chain protection groups were t-butyl for serine and tyrosine hydroxyls, trityl for glutamine amides, and Pmc (2,2,5,7,8-pentamethylchroman sulfonate) for the arginine guanidino group. Each coupling was performed for either one or two hours with BOP (benzotriazolyl N-oxtrisdimethylaminophosphonium hexafluorophosphate) and HOBt (1-hydroxybenztriazole).

In the synthesis of peptides with an amidated carboxy terminus, the fully assembled peptide was cleaved with a mixture of 90% trifluoroacetic acid; 5% ethanedithiol, and 5% water, initially at 4°C, and gradually increasing to room temperature over 1.5 hours. The deprotected product was filtered from the resin and precipitated with diethyl ether. After thorough drying the product was purified by C18 reverse phase high performance liquid chromatography with a gradient of acetonitrile/water in 0.1% trifluoroacetic acid.

EXAMPLE 2

Bioassays

***A. FDCP-1/mEPO-R***

The FDCP-1/mEPO-R cells ($10^5$) were grown to half stationary density in the presence of growth factors (2.5 nm EPO). The cells were washed twice in PBS and then starved for 24 hours in whole media minus growth factors.

Cell viability was determined by trypan blue staining. Stock in whole media minus growth factors was made to give desired number of cells per 50 μl. The compounds to be tested were diluted 2-fold in 96 well tissue culture plate (50 μl per well final).

Cells (50 μl per well) were added to each well and incubated 24-48 hours (when the negative controls should die). Cell proliferation was determined by MTT assay.

***B. TF-1***

The procedure set forth in Kitamura *et al.* (*Blood 73*:375-380 (1989), which is incorporated herein by reference) relating to the cell line TF-1 having growth dependency on EPO was also conducted to demonstrate the activities of the compounds of the present invention as EPO agonists. Representative results are shown in Figure 8. Figure 8 depicts the effects of EPO and the peptide VGNYMCHFGPITWVCRPGGG on cellular proliferation of the cell line TF-1.

***C. Spleen Cell Proliferation***

The procedure set forth in Krystal (*Exp. Hematol. 11*:649-660 (1983), which is incorporated herein by reference) for a microassay based on $^3$H-thymidine incorporation into spleen cells was also employed to ascertain the ability of the compounds of the present invention to serve as EPO agonists. In brief, B6C3F$_1$ mice were injected daily for two days with phenylhydrazine (60 mg/kg). On the third day, spleen cells were removed and their ability to proliferate over 24 hours using an MTT assay was ascertained. Photographs showing the proliferation of representative populations of spleen cells are shown in Figures 9A (control), 9B (treated with 500 pM EPO), and 9C (treated with 25 pm GGDYHCRMGPLTWVCKPLGG) at 200 X magnification.

***D. Cellular Proliferation: Peptide Dependent Growth of the EPO-Responsive Cell Line, FDC-P1/ER***

1. Materials and Method

a. Sample Preparation: Peptides were resuspended in DMSO as a 1 x $10^{-2}$ M stock solution and serially diluted in 10-fold increments to generate the following 100X solutions:

1 x $10^{-3}$ 1 x $10^{-4}$ 1 x $10^{-5}$ 1 x $10^{-6}$ 1 x $10^{-7}$ 1 x $10^{-8}$

Two µl of each stock dilution was added to a cell well as described below in a total volume of 200 µl to generate final concentrations as follows:

1 x $10^{-5}$ 1 x $10^{-6}$ 1 x $10^{-7}$ 1 x $10^{-8}$ 1 x $10^{-9}$ 1 x $10^{-10}$

b. Cell Proliferation Assay:

i. Cell source: FDC-P1/ER, (Dexter, *et al*., *J. Exp. Med*. (1980) 152:1036-1047, which is incorporated herein by reference), is a well characterized nontransformed murine bone marrow derived cell line in which the erythropoietin receptor has been stably transfected. Cells exhibit EPO dependent proliferation.

c. Experimental Protocol: Cells maintained in RPMI 1640/10% Fetal Bovine Serum/antibiotics and 10 U/ml erythropoietin are grown to stationary phase. Cells are harvested and resuspended in fresh medium without growth factor (erythropoietin) for 24 hr. Cells are counted and resuspended at a concentration of 800,000 cells/ml. Approximately forty thousand cells (50µ) are added to each well of a 96 well microtiter plate. Peptide is added to each well in triplicate. A standard dose response determination is run with each series of peptides. After a forty-two hr incubation (≈ 2 doubling), each well is pulsed with 1 µCi/well Thymidine. Cells are incubated an additional six hr at which time cells are harvested and counted to assess thymidine incorporation as an indicator of cell proliferation.

d. Results: Peptides are evaluated on both the erythropoietin receptor cell line and the parental nonreceptor bearing cell line. In most cases, the peptide has been evaluated on a truncated human erythropoietin receptor cell line. Results are expressed as the amount of peptide necessary to yield one half of the maximal activity obtained with recombinant erythropoietin. Representative results are reported in tabular form with the relative binding data *(See,* Tables 17-22, *infra*.)

***E. Tyrosine Phosphorylation: Peptide Induced Tyrosine Phosphorylation of Erythropoietin Receptor and Intracellular Proteins***

1. Materials and Method

a. Sample preparation: Peptide was resuspended in DMSO to yield a concentrate of 1 x $10^{-2}$ M.

b. Experimental Protocol: FDC-P1/muER cells maintained in RPMI 1640/10% Fetal Bovine Serum/antibiotics and 10 U/ml erythropoietin are grown to stationary phase. Cells are harvested and resuspended in fresh medium without growth factor (erythropoietin) for 24 hr. Cells are counted and resuspended at a concentration of 500,000 cells/ml. One milliliter of cells (500,000) are placed in an eppendorf tube. One microliter of a 1 x $10^{-3}$ peptide solution is added to the cells (final concentration 1 x $10^{-5}$ M) and allowed to incubate for 10 minutes at 37°C. An erythropoietin control (final concentration 10 U/ml) was run with each assay. Cells are collected by centrifugation at 14,000 rpm 4°C. Cells are resuspended in 100 µl of SDS lysis buffer and subjected to SDS polyacrylamide gel electrophoresis. The gel is transferred to nitrocellulose and probed with an anti-phosphotyrosine antibody (Upstate Biotechnology Incorporated) diluted 1:1000 for 1 hour. The membrane is washed with Tris buffered saline and reprobed with an anti-mouse peroxidase labeled antibody. Reactive proteins are visualized using the ECL western blotting reagents (Amersham).

c. Results: Erythropoietin binding to its receptor in an erythropoietin-responsive cell line induces tyrosine phosphorylation of both the receptor and numerous intracellular proteins, including Shc, vav and JAK2 kinase. Numerous peptides, including GGTYSCHFGPLTWVCKPQGG, have been analyzed to assess their ability to mimic the response seen with erythropoietin. Active peptides, as judged by binding and proliferation criteria, elicit an identical phosphorylation pattern as that of erythropoietin in erythropoietin-responsive cells. These results implicate that active peptides elicit their response through an erythropoietin-induced signal transduction pathway. Results are reported in tabular form with the binding and proliferation data (*See,* Tables 17-22, *infra*).

***F. Cellular response kinetics: Peptide-Induced Inibiation of Cell Cycle Measured By DNA Content***

1. Experimental Protocol: FDCP-1/muER cells were grown to stationery phase ≈ 3.0 x $10^6$ cells/ml. The cells were collected and resuspended in fresh medium in the absence of factor and allowed to grow for an additional 18 hr at which point the cells were divided into three flasks of equal cell density: -factor, +EPO and +peptide. Cells were then stimulated with either 10 U/ml EPO or 10 μM peptide. Three million cells were collected at the following time points: 0, 6, 8, 10, and 12 hr. *(see,* Figure 15). Cells were washed twice in cold PBS and fixed by resuspending the cell pellet in 100 μl of cold PBS followed by 900 μl of cold 70% ethanol. Cells were stained with 50 μg/ml of propidium iodine and fluorescence was measured on FACS Scan Flow cytometer. The percentage of cells in each phase was measured using the SOBR model of CellFIT software, by Becton Dickinson.

Erythropoietin (10 U/ml) and GGTYSCHFGPLTWVCKPQGG (1 x $10^{-5}$ M) are equally effective at progressing cells through the cell cycle. By ten hours postinduction by either erythropoietin or peptide, approximately 45% of cells are in S phase as compared to the medium control of 15%. This result suggests that the peptide is able to elicit its response with the same kinetics as recombinant erythropoietin.

***G. Colony Assay: Murine Bone Marrow and Human Peripheral Blood Colony Assay***

1. Materials and Methods:

A ten milliliter sample of peripheral blood was obtained from a healthy individual in a standard sterile heparin vacutainer tube. Murine bone marrow was obtained from the femurs of approximately 10 mice per experiment. All reagents were obtained from Stem Cell Technologies Inc. (Vancouver, Canada).

a. Experimental Protocol: Briefly, a nucleated cell count was performed to establish the number and concentration of nucleated cells in the original sample. In the case of peripheral blood, the sample is subjected to centrifugation through a Ficoll-Hypaque (1.077 g per ml) gradient at 400 g for 30 minutes at room temperature. Mononuclear cells at the interface of the Ficoll-Hypaque solution are carefully removed and diluted to a total volume of about 10 ml. The cells obtained from murine bone marrow or human peripheral blood were collected by centrifugation and the supernatant was decanted. The pelleted cells were resuspended in fresh medium and subjected to collection as described above. The washed cells were diluted in Iscove's medium/2% fetal bovine serum to the following concentration for plating:

| | |
|---|---|
| Normal marrow | 1 x $10^5$ light density cells |
| Normal blood | 4 x $10^5$ light density cells |

Cells were added to methyl cellulose per directions of the manufacturer. Peptides were assayed at the following final concentrations: 1 x 10-6 and 1 x 10-5 M in a "minus EPO" methylcellulose medium. Equivalent cell numbers were plated in "Complete" methylcellulose medium to assess maximal colony formation. A control plate was run with each assay minus EPO and peptide to determine background colony formation. Colonies were scored after both 10 days and 18 days of incubation.

b. Results: As shown in Tables 13-15, GGTYSCHPGPLTWVCKPQGG was able to promote colony formation, albeit at significantly lower levels as compared to that obtained on "Complete" methylcellulose medium (Erythropoietin 3 U/ml), in both murine bone marrow and human peripheral blood.

Table 13.

| COLONY ASSAY ON A HUMAN PERIPHERAL BLOOD SPECIMEN (DONOR: MALE) (SET UP DATE: 2/10/94) | | | | | |
|---|---|---|---|---|---|
| DATE | SAMPLE | CFU-E | MBFU-E | PBFU-E | CFU-GM |
| 2/23/94 | ZERO | X | X | X | X |
| | DMSO | X | X | X | X |
| | EPO(METHOCULT) | 11 | 25 | X | X |
| | EPO (3U/ml) | 7 | 7 | X | X |
| | AFFY 244 $10^{-5}$M | 10 | 5 | X | X |
| | AFFY 244 $10^{-6}$M | 11 | 5 | X | X |
| 3/4/94 | | | | | |
| | ZERO | X | X | X | X |
| | DMSO | X | X | X | X |
| | EPO(METHOCULT) | 1 | 11 | 13 | 9 |
| | EPO(3U/ml) | 4 | 7 | 7 | 3 |
| | AFFY 244 $10^{-5}$M | 7 | 9 | 2 | 4 |
| | AFFY 244 $10^{-6}$M | 6 | 11 | 1 | 2 |
| CFU-E: COLONY-FORMING UNIT-ERYTHROID (1-2 CLUSTERS)<br>MBFU-E: MATURE BURST-FORMING UNIT-ERYTHROID (3-8 CLUSTERS)<br>PBRU-E: PRIMITIVE BURST-FORMING UNIT-ERYTHROID (9 OR MORE)<br>CFU-GM: COLONY-FORMING UNIT GRANULOCYTIC/MONOCYTE/MACROPHAGE | | | | | |

Table 14.

| Colony-Forming Cell Numbers (Murine Bone Marrow) | | | | | |
|---|---|---|---|---|---|
| 1/28/94 | | | | | |
| | 2/10/94 | | | AFFY11157 / AFFY 244 | |
| | Progenitor | Control | EPO | $10^{-5}$M | $10^{-6}$M |
| | CFU-E | 0 | 23 | 2 | 3 |
| | BFU-E | 0 | 5 | 0 | 0 |
| | CFU-GM | 0 | 3 | 0 | 0 |
| | 2/21/94 | | | AFFY11157 / AFFY 244 | |
| | Progenitor | Control | EPO | $10^{-5}$M | $10^{-6}$M |
| | CFU-E | 0 | 0 | 0 | 0 |
| | BFU-E | 0 | 30 | 2 | 4 |
| | CFU-GM | 0 | 3 | 0 | 0 |
| AFFY11157 = ggTYSCHFGPLTWVCKPQgg | | | | | |

Table 15.

| Colony-Forming Cell Numbers (Murine Bone Marrow) | | | | | |
|---|---|---|---|---|---|
| 2/21/94 | | | | | |
| | 3/1/94 | | | AFFY11157 / AFFY 244 | |
| | Progenitor | Control | EPO | $10^{-5}$M | $10^{-6}$M |
| | CFU-E | 0 | 3 | 9 | 0 |
| | BFU-E | 0 | 3 | 12 | 0 |
| | CFU-GM | 0 | >10 | 0 | 0 |

Table 15. (continued)

| Colony-Forming Cell Numbers (Murine Bone Marrow) | | | | | |
|---|---|---|---|---|---|
| | 3/14/94 | | | AFFY11157 / AFFY 244 | |
| | Progenitor | Control | EPO | $10^{-5}$M | $10^{-6}$M |
| | CFU-E | 0 | 0 | 1 | 0 |
| | PBFU-E | 0 | 10 | 8 | 0 |
| | MBFU-E | 0 | 0 | 4 | 0 |
| | CFU-GM | 0 | 26 | 0 | 0 |

*H. Peptide-Induced Proliferation: Peptide-Dependent Growth on Cell Lines Not Responsive to Erythropoietin*

a. Experimental protocol: Peptides were assayed as described in Section D, *supra*. A growth curve was performed on each cell line using the relevant mitogen/growth factor for each cell line to assess growth potential.

b. Results: As shown in Table 16, GGTYSCHFGPLTWVCKPQGG was unable to stimulate a growth response on non-erythropoietin responsive cell lines including both hematopoietic and non-hematopoietic cell lines.

Table 16.

| Cell Proliferation Assays with 61233 | | | | |
|---|---|---|---|---|
| **Cell type** | **Species** | **EPO** | **61233** | **Growth factor response** |
| hematopoietic | | | | |
| TF-1 | human | + | + | GM-CSF, IL3 (erythroid) |
| M-07E | human | - | - | GM-CSF, IL3 (megakaryoblastic) |
| AML193 | human | - | - | GM-CSF, G-CSF, $IL_3$ (monocytic) |
| T-cell clone | human | ND | - | IL2 |
| osteoblastic | | | | |
| TE85 | human | - | - | serum |
| MC3T3 | murine | - | - | serum |
| breast cell | | | | |
| T47D | human | ND | - | progestin |

*I. Immobilized EBP Based [$^{125}$I]EPO Competition Binding Assay.*

The extracellular domain of the human erythropoietin receptor (EPO binding protein, EBP) has been expressed and overproduced in *E. coli.* As with many other recombinant eukaryotic proteins used in *E. coli,* the protein appeared as an insoluble product in laboratory scale fermentations and was refolded and purified to obtain active protein. EBP as produced by this method contains one free sulfhydryl group which can be modified without effecting the solution phase binding of ligand. In order to immobilize the EBP for equilibrium binding analysis and as the basis for a competition binding assay, this observation was extended for the covalent attachment of EBP to agarose beads. The iodoacetyl activation chemistry of Sufolink beads (Pierce Chemical Co, Rockford, IL) is specific for free thiols and assures that the linkage is not easily reversible. EBP-Sulfolink beads were made as follows: SulfoLink gel suspension (10 ml) was mixed with coupling buffer (40 ml: 50 mM Tris, pH 8.3, 5 mM EDTA) and the gel was allowed to settle. The supernatant was removed and the EBP (0.3-1 mg/ml in coupling buffer) to be bound was added directly to the washed beads. The mixture was rocked gently for 30 minutes at room temperature, and the beads were allowed to settle for 1 hour at room temperature. The supernatant was removed and retained, and the beads were washed twice with 20 ml of coupling buffer. The washes were recovered as well. The beads were then treated with 20 ml of 0.05 M cysteine for 30 minutes at room temperature to block unbound sites. Finally, the beads were washed with 50 ml of 1 M NaCl, then with 30 ml of PBS, and resuspended in 20 ml of PBS and stored at 4°C for later use. The amount of EBP which was covalently bound to the beads was determined by comparing the $OD_{280}$ of the original EBP solution to the total $OD_{280}$ recovered in the reaction supernatant and the two 20 ml washes. Typically, 40-60% of the applied EBP remains associated with the beads.

Binding assays were initiated by the addition of EBP beads (50 µl) to individual reaction tubes. Total binding was measured in tubes containing 0.3-30 nM $^{125}$I-EPO (NEN Research Products, Boston MA, 100 µCi/µg). For determination of non-specific binding, unlabelled EPO was added at a level of 1000 fold in excess of the corresponding [$^{125}$I]EPO concentration. Each reaction volume was brought to 500 µl with binding buffer (PBS/0.2% BSA). The tubes were incubated for five hours (a time period experimentally determined as adequate for the establishment of equilibrium) at room temperature with gentle rocking. After five hours, each reaction mixture was passed through a 1 ml pipet tip plugged with glass wool. The tubes were washed with 1 ml wash buffer (PBS/5% BSA) and this volume, as well as 2 additional 1 ml washes, were passed through the pipet tip and collected for determination of the free EPO concentration. Equilibrium binding analysis of the specific association of [$^{125}$I]EPO with EBP immobilized on these agarose beads indicates a Kd of 5 nM ± 2 based on a linear transformation (Scatchard) of the binding isotherm *(see,* Figure 16).

Competitive binding analysis assays of candidate peptides were performed as outlined below. Individual peptides were dissolved in DMSO to prepare a stock solution of 1 mM. All reaction tubes (in duplicate) contained 50 µL of EBP beads, 0.5 nM [$^{125}$I]EPO and 0-500 µM peptide in a total of 500 µL binding buffer. The final concentration of DMSO was adjusted to 2.5 % in all assay tubes, a value without detectable effect since an examination of the sensitivity of the assay to DMSO demonstrated that concentrations of up to 25% DMSO (V/V) had no deleterious effect on binding. Non-specific binding was measured in each individual assay by inclusion of tubes containing a large excess of unlabelled EPO (1000 nM). Initial assay points with no added peptide were included in each assay to determine total binding. Binding mixtures were incubated overnight at room temperature with gentle rocking. The beads were then collected using Micro-columns (Isolab, Inc., Akron, Ohio) and washed with 3 mL of wash buffer. The columns containing the washed beads were placed in 12 x 75 mm glass tubes and bound radioactivity levels were determined using a gamma counter. The amount of bound [$^{125}$I]EPO was expressed as a percentage of the control (total = 100 %) binding and plotted versus the peptide concentration after correction for non-specific binding. The $IC_{50}$ was defined as the concentration of peptide which reduced the binding of [$^{125}$I]EPO to the EBP beads by 50%. All data are reported as relative to GGTYSCHFGPLTWVCKPQGG (RWJ61233) which demonstrated an $IC_{50}$ of 5 µM (*see,* Tables 17-22, *infra*).

**Table 17. GGTYSCHFGPLTWVCKPQGG Substitution Series**

| SEQ ID NO. | Sequence | Relative Binding[‡] | EPO-$ED_{50}$ (µM) | Phosphorylation[1] |
|---|---|---|---|---|
| 61233[‡] | GGTYSCHFGPLTWVCKPQGG | 1 | 0.1 | + |
| 61231 | GGTASCHFGPLTWVCKPQGG | 24 | IA | - |
| 61520 | GGTTSCHFGPLTWVCKPQGG | 24 | IA | - |
| 61598 | GGTFSCHFGPLTWVCKPQGG | 12 | IA | - |
| 61277 | GGTYSCHFGALTWVCKPQGG | 2 | 0.1 | + |
| 61278 | GGTYSCHFGPLAWVCKPQGG | 18 | IA | - |
| 61313 | GGTYSCHFAPLTWVCKPQGG | 16 | IA | - |
| 61314 | GGTYSCHFGPATWVCKPQGG | 1 | .2 | + |
| 61395 | GGTYSCHFGPLTAVCKPQGG | >100 | IA | - |
| 62145 | GGTYSCHFGPLTFVCKPQGG | 6 | 0.25 | |
| 61530 | GGTYSC-FGPLTWVCKPQGG | 40 | IA | - |

[*] Amount required to achieve the half maximal level of EPO dependent proliferation (11pM)
[1] Assayed at 10 µM
[2] IA = inactive
[‡] Binding relative to RWJ-61233
[‡] Note that all peptides are cyclic (except 61394) and were analyzed as COOH terminal amides ($-CONH_2$)

61233 = AFFY11157 = AFFY 244

**Table 18. GGTYSCHFGPLTWVCKPQGG Truncation Series**

| RWJ No. | Sequence | Relative Binding‡ | EPO-$ED_{50}$ ($\mu M$) | Phosphorylation[1] |
|---|---|---|---|---|
| 61233‡ | GGTYSCHFGPLTWVCKPQGG | 1 | 0.1 | + |
| 61596 | GGTYSCHFGPLTWVCKPQ | 1.6 | 0.08 | + |
| 61597 | TYSCHFGPLTWVCKPQGG | 8 | 2 | + |
| 61230 | TYSCHFGPLTWVCKPQ | 6 | 2 | + |
| 61232 | YSCHFGPLTWVCKP | 9 | 20 | +/- |
| 61279 | YSCHFGPLTWVCK | 14 | 3 | +/- |
| 61477 | YSCHFGPLTWVC | 50 | IA[2] | - |
| 61895 | YSCHFGALTWVCK | 32 | IA | |
| 61513 | Y-CHFGPLTWVC | 12 | 2 | + |
| 61177 | SCHFGPLTWVCK | 18 | IA | - |
| AF11154 | GGCRIGPITWVCGG | 13 | IA | - |
| 61394 | HFGPLTWV | >100 | IA | - |

* Amount required to achieve the half maximal level of EPO dependent proliferation (11pM)
[1] Assayed at 10 $\mu M$
[2] IA = inactive
‡ Binding relative to RWJ-61233
‡ Note that all peptides are cyclic (except 61394) and were analyzed as COOH terminal amides ($-CONH_2$)

61233 = AFFY11157 = AFFY244

**Table 19. Sequence Analog Peptides**

| RWJ No. | Sequence | Relative Binding[‡] | EPO-$ED_{50}$ ($\mu M$) | Phosphorylation[1] |
|---|---|---|---|---|
| 61233[‡] | GGTYSCHFGPLTWVCKPQGG | 1 | 0.1 | + |
| 61721 | GGTYSEHFGPLTWVKKPQGG | >100 | IA[2] | |
| 61718 | GGLYACHMGPMTWVCQPLRG | 0.6 | 0.1 | |
| 61719 | GGEYLCRMGPMTWVCTPVGG | 8 | 9 | |
| 61720 | GGLYTCRMGPITWVCLPAGG | ND | - | |
| 61717 | GGNYYCRFGPITFECHPTGG | >100 (S1) | IA | |

[*] Amount required to achieve the half maximal level of EPO dependent proliferation (11pM)
[1] Assayed at 10 $\mu M$; [2]IA = inactive
[‡] Binding relative to RWJ-61233
[‡] Note that all peptides are cyclic (except 61394) and were analyzed as COOH terminal amides ($-CONH_2$)

61233 = AFFY11157 = AFFY244

**Table 20. Position #4 Substitution Series**

| RWJ No. | (X) | Sequence | Relative Binding[‡] | EPO-$ED_{50}$ ($\mu M$)* | |
|---|---|---|---|---|---|
| | | | | murine receptor | truncated human receptor |
| 61233[‡] | | GGTYSCHFGPLTWVCKPQGG | 1 | 0.1 | 0.1 |
| 61231 | | GGTASCHFGPLTWVCKPQGG | 24 | IA[2] | |
| 61520 | | GGTTSCHFGPLTWVCKPQGG | 24 | IA | |
| 61598 | | GGTFSCHFGPLTWVCKPQGG | 12 | IA | |
| 61894 | p-$NO_2$-Phe | GGTXSCHFGPLTWVCKPQGG | 17 | IA | 0.8 |
| 62019 | p-$NO_2$-Phe | GGTXSCHFGPLTWVCKPQGG | 18 | IA | 3.0 |
| 62020 | p-F-Phe | GGTXSCHFGPLTWVCKPQGG | 8 | 1.0 | 0.1 |
| 62021 | 3,5-dibromo-tyr | GGTXSCHFGPLTWVCKPQGG | 30 | IA | IA |

* Amount required to achieve the half maximal level of EPO dependent proliferation (11pM)
[1] ND = Not determined
[2] IA = Inactive
[‡] Binding relative to RWJ-61233

Note that all peptides are cyclic and were analyzed as COOH terminal amides ($-CONH_2$)

61233 = AFFY11157 = AFFY244

**Table 21. Position #17 Substitution Series**

| Sequence No. | Sequence | Relative Binding** | EPO-ED$_{50}$ (μM) | | |
|---|---|---|---|---|---|
| | | | murine receptor | truncated human receptor | human receptor |
| 65876 | GGTYSCHFGPLTWVCKAQGG | >20* | 10 | 0.3 | 10 |

** Binding relative to 61233 = AFFY11157 = AFFY244
* Solubility limited

**Table 22. Differential Activity Series-GGTYSCHFGPLTWVCKPQGG Based**

| RWJ No. | Sequence | Relative Binding‡ | EPO-$ED_{50}$ ($\mu M$)* murine receptor | truncated human receptor |
|---|---|---|---|---|
| 61233‡ | GGTYSCHFGPLTWVCKPQGG | 1 | 0.1 | 0.1 |
| 61596 | GGTYSCHFGPLTWVCKPQ | 1.6 | 0.08 | 0.02 |
| 61718 | GGLYACHMGPMTWVCQPLRG | 0.6 | 0.1 | 0.08 |
| AF11288 | GGLYACHMGPMTWVCQPLRG | 0.2 | 0.15 | ND[1] |
| 61757 (H22) | LGRKYSCHFGPLTWVCQPAKKD | 1 | 0.11 | 0.15 |
| AF11654 | TIAQYICYMGPETWECRPSPKA | 1 | 0.2 | 0.02 |
| 62145 | GGTYSCHFGPLTFVCKPQGG | 6 | 0.25 | 0.5 |
| 62146 | Ac-GGTYSCHFGPLTWVCKPQGG | 4 | 0.03 | 0.06 |
| 61894 | GGTXSCHFGPLTWVCKPQGG p-$NO_2$-Phe | 17 | IA[2] | 0.8 |
| 62019 | GGTXSCHFGPLTWVCKPQGG p-$NH_2$-Phe | 18 | IA | 3.0 |
| 62020 | GGTXSCHFGPLTWVCKPQGG p-F-Phe | 8 | 1.0 | 0.1 |

* Amount required to achieve the half maximal level of EPO dependent proliferation (11pM)
[1] ND = Not determined
[2] IA = Inactive
‡ Binding relative to RWJ-61233
Note that all peptides are cyclic and were analyzed as COOH terminal amides ($-CONH_2$)

61233 = AFFY11157 = AFFY244

*J. Polycythemic Exhypoxic Mouse Bioossay*

Female BDF1 mice (18-20 gms) are subjected to a conditioning cycle (in hypobaric chambers) of 18 hrs. at 0.40 ± 0.02 atm. and 6 hrs. at ambient pressure for a period of 14 days.

Mice are maintained at ambient pressure for 72 hrs prior to administration of r-HuEPO or test samples. Test samples or r-HuEPO standard are diluted for injection into conditioned mice. The vehicle consists of: PBS containing 0.1 % BSA (w/v). For each dilution, 0.5 ml is administered subcutaneously into each of 10 mice. $^{59}Fe$ is administered

48 hrs. later. Blood samples are taken 48 hrs. following administration of $^{59}Fe$. Hematocrits and radioactivity measurements are determined for each sample. Dose range and results (two different assays) are set forth in Table 23 and Figures 17A-17C, *infra*.

Table 23.

| | DOSE | n | LOG MEAN |
|---|---|---|---|
| EPO St. | 0.000 U | 10 | 0.26 |
| | 0.025 | 10 | 1.65 |
| | 0.050 | 10 | 2.74 |
| | 0.100 | 10 | 3.28 |
| DMSO | 1.40% | 7 | 0.58 |
| | 0.35% | 5 | 0.60 |
| 244-1 | 0.25 µg | 7 | 0.62 |
| | 0.50 | 8 | 0.76 |
| | 1.00 | 9 | 0.92 |
| 244-2 | 2.00 | 9 | 1.38 |
| | 4.00 | 10 | 1.81 |
| | 8.00 | 9 | 2.26 |
| 244-3 | 14.0 | 10 | 2.44 |
| | 28.0 | 10 | 3.15 |
| | 56.0 | 9 | 3.16 |
| 0.1 mg AFFY244 ≈ 1 U r-HuEPO (8.3 ng) | | | |

In addition, using the foregoing polycythemic exhypoxic mouse bioassay, TIAQYICYMGPETWECRPSPKA and a dimeric peptide analog of GGTYSCHFGPLTWVCKPQGG containing two disulfide bonds (AF12080) were tested. The results of the polycythemic exhypoxic mouse bioassay are set forth in Figures 18A-18B. In addition, Table 24, *infra*, sets forth the approximate equivalency of EPO and various mimetic peptides.

**Table 24.**

| Peptide | Amount of Peptide Equivalent to 0.025 units of EPO (nmol) |
|---|---|
| GGTYSCHFGPLTWVCKPQGG (RWJ 61233/AFFY11157) | 3.8 |
| GGTYSCHFGPLTWVCKPQ (RWJ 61596) | 0.5 |
| LGRKYSCHFGPLTWVCQPAKKD (RWJ 61757) | 3.1 |
| TIAQYICYMGPETWECRPSPK (AAFY11654) | 11-21 |
| GGTYSCHFGPLTWVCKPQGG Dimer (AFFY12080) | 0.035 |

*K. Reticulocyte Assay*

Normal untreated female BDF1 mice are dosed (0.5 ml, S.Q.) on three consecutive days with either EPO or experimental compound. The vehicle: PBS, 10% PEG 8000, 0.25% BSA, with DMSO being added. At day three, mice are also dosed (0.1 ml, I.P.) with iron dextran (100 mg/ml). At day five, mice are anesthetized with $CO_2$ and bled by

cardiac puncture. % reticulocytes is determined by thiazole orange staining and flow cytometer analysis (retic-count program). Hematocrits are manually determined. The percent of corrected reticulocytes is determined using the following formula:

$$\% \text{ RETIC }_{(CORRECTED)} = \% \text{ RETIC.}_{(OBSERVED)} \text{ X Hct.}_{(INDIVIDUAL)}/\text{Hct.}_{(NORMAL)}.$$

The results obtained using the foregoing reticulocyte assay are set forth in Figures 18A-18B and Figures 19A-19B.

### *L. Colony Assay: Erythroid Colony Formation*

#### 1. Materials and Methods:

Human nucleated bone marrow cells obtained from normal donors were plated in semi-solid methylcellulose (0.9% methylcellulose, 30% fetal bovine serum, 1% bovine serum albumin, $10^{-4}$ 2-mercaptoethanol, 2 mM L-glutamine). Briefly, cells are collected and red cells are lyzed by the addition of buffered ammonium chloride. Cells were resuspended in medium containing 2 % fetal calf serum and plated at a density of 2 x $10^5$ cells per plate in duplicate. Erythroid and granulocyte-macrophage (G-M) colonies formed after 12 days in culture were scored by color and morphology. A control plate was included in study containing recombinant human growth factors IL-3, GM-CSF, and SCF (3/GM/S) to access GM colony formation of sample.

| Growth Factor (nM) | Number of Colonies per 2 x $10^5$ nucleated cells | | | |
|---|---|---|---|---|
| | Erythroid | | G-M | |
| | Plate 1 | Plate 2 | Plate 1 | Plate 2 |
| Control (no factors) | 0 | 0 | 0 | 0 |
| EPO (0.24) | 113 | 106 | 12 | 9 |
| RWJ 61233 (1000) | 29 | 23 | 1 | 2 |
| RWJ 61233 (10,000) | 80 | 75 | 2 | 0 |
| 3/GM/S (0.7/0.7/2.7) | 0 | 0 | 176 | 212 |

#### 2. Results:

From the data set forth above, it is apparent that GGTYSCHFGPLTWVCKPQGG (RWJ 61233/AFFY11157) is able to promote erythroid colony formation in the absence of additional cytokines. In addition, the peptide exhibits strict specificity for the erythroid lineage by its inability to promote colony formation of the GM lineage.

It is to be understood that the above description is intended to be illustrative and not restrictive. Many embodiments will be apparent to those of skill in the art upon reviewing the above description. The scope of the invention should, therefore, be determined not with reference to the above description, but should instead be determined with reference to the appended claims, along with the full scope of equivalents to which such claims are entitled. The disclosures of all articles and references, including patent publications, are incorporated herein by reference.

1. A peptide of 10 to 40 amino acid residues in length that binds to erythropoietin receptor and comprises a sequence of amino acids $X_3X_4X_5GPX_6TWX_7X_8$ where each amino acid is indicated by standard one letter abbreviation; $X_6$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**2.** The peptide of paragraph **1** that comprises a sequence of amino acids $YX_2X_3X_4X_5GPX_6TWX_7X_8$ where each amino acid is indicated by standard one letter abbreviation; each of $X_2$ and $X_6$ is independently selected from any one of the 20 genetically coded L-amino acids; $X_3$ is C; $X_4$ is R, H, L, or W; $X_5$ is M, F, or I; X7 is D, E, I, L, or V; and $X_8$ is C.

**3.** The peptide of paragraph **2** that comprises a sequence of amino acids $X_1YX_2X_3X_4X_5GPX_6TWX_7X_8X_9X_{10}X_{11}$, where each amino acid is indicated by standard one letter abbreviation; each of $X_1$, $X_2$, $X_6$, $X_9$, $X_{10}$, and $X_{11}$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**4.** The peptide of paragraph **3** wherein $X_4$ is R or H; $X_5$ is F or M; $X_6$ is I, L, T, M, E, or V; $X_7$ is D or V; $X_9$ is G, K, L, Q, R, S, or T; and $X_{10}$ is A, G, P, R, or Y.

**5.** The peptide of paragraph **4** wherein $X_1$ is D, E, L, N, S, T; or V; $X_2$ is A, H, K, L, M, S, or T; $X_4$ is R or H; $X_9$ is K, R, S, or T; and $X_{10}$ is P.

**6.** The peptide of paragraph **1** that is selected from the group consisting of:

GGLYLCRFGPVTWDCGYKGG;
GGTYSCHFGPLTWVCKPQGG;
GGTYSCHFGPLTWVCKPQ;
GGDYHCRMGPLTWVCKPLGG;
VGNYMCHFGPITWVCRPGGG;

GGNYMCHFGPITWVCRPGGG;
GGVYACRMGPITWVCSPLGG;
VGNYMAHMGPITWVCRPGG;
GGPHHVYACRMGPLTWIC;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
SCHFGPLTWVCK;
GGTYSCFGPLTWVCKPQGG;
TYSCHFGPLTWVCKPQGG;
YSCHFGPLTWVC;
GGTYSCHFGPLTFVCKPQGG; and
HFGPLTWV.

**7.** The peptide of paragraph **1** that is selected from the group consisting of:

GGLYLCRFGPVTWDCGYKGG;
GGTYSCHFGPLTWVCKPQGG;
GGDYHCRMGPLTWVCKPLGG;
VGNYMCHFGPITWVCRPGGG;
GGVYACRMGPITWVCSPLGG;
VGNYMAHMGPITWVCRPGG;
GGPHHVYACRMGPLTWIC;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC; and
HFGPLTWV.

**8.** The peptide of paragraph **1** wherein said sequence of amino acids is cyclized.

**9.** The peptide of paragraph **8** wherein said peptide is selected from the group consisting of

GGTYSCHFGPLTWVCKPQGG;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
YSCHFGALTWVCK;
SCHFGPLTWVCK;
GGTYSEHFGPLTWVKKPQGG;
GGTYSCFGPLTWVCKPQGG;
TYSCHFGPLTWVCKPQGG;
YSCHFGPLTWVC;
GGTYSCHFGPLTFVCKPQGG; and
GGTYSEHFGPLTWVKKPQGG.

**10.** The peptide of paragraph **1** wherein said sequence of amino acids is dimerized.

**11.** The peptide of paragraph **10** wherein said peptide comprises the following amino acid sequence:

**GGTYSCHFGPLTWVCKPQGG**

**GGTYSCHFGPLTWVCKPQGG.**

**12.** A pharmaceutical composition comprising a compound of paragraph **1** in combination with a pharmaceutically acceptable carrier.

**13.** A method for treating a patient having a disorder characterized by a deficiency of erythropoietin or a low or defective red blood cell population comprising administering to the patient a therapeutically effective amount of a peptide of paragraph **1**.

**14.** The method of paragraph **13** wherein the disorder is end-stage renal failure or dialysis; anemia associated with AIDS, autoimmune disease, or malignancies; beta-thalassemia; cystic fibrosis; early anemia of prematurity; anemia associated with chronic inflammatory diseases; spinal cord injury; acute blood loss; aging; and neoplastic disease states accompanied by abnormal erythropoiesis.

**15.** The method of paragraph **13** wherein the peptide comprises a sequence of amino acids $YX_2X_3X_4X_5GPX_6TWX_7X_8$ where each amino acid is indicated by standard one letter abbreviation; each $X_2$ and $X_6$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**16.** The method of paragraph **15** wherein the peptide comprises a sequence of amino acids $X_1YX_2X_3X_4X_5GPX_6TWX_7X_8X_9X_{10}X_{11}$, where each amino acid is indicated by standard one letter abbreviation; each $X_1$, $X_2$, $X_6$, $X_9$, $X_{10}$, and $X_{11}$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**17.** The method of paragraph **16** wherein $X_4$ is R or H; $X_5$ is F or M; $X_6$ is I, L, T, M, or V; $X_7$ is D or V; $X_9$ is G, K, L, Q, R, S, or T; and $X_{10}$ is A, G, P, R, or Y.

**18.** The method of paragraph **17** wherein $X_1$ is D, E, L, N, S, T, or V; $X_2$ is A, H, K, L, M, S, or T; $X_4$ is R or H; $X_9$ is K, R, S, or T; and $X_{10}$ is P.

**19.** The method of paragraph **13** wherein the peptide is selected from the group consisting of:

**GGLYLCRFGPVTWDCGYKGG;**
**GGTYSCHFGPLTWVCKPQGG;**
**GGTYSCHFGPLTWVCKPQ;**
**GGDYHCRMGPLTWVCKPLGG;**
**VGNYMCHFGPITWVCRPGGG;**

GGNYMCHFGPITWVCRPGGG;
GGVYACRMGPITWVCSPLGG;
VGNYMAHMGPITWVCRPGG;
GGPHHVYACRMGPLTWIC;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
SCHFGPLTWVCK;
GGTYSCFGPLTWVCKPQGG;
TYSCHFGPLTWVCKPQGG;
YSCHFGPLTWVC;
GGTYSCHFGPLTFVCKPQGG; and
HFGPLTWV.

**20.** The method of paragraph **13** wherein the peptide is cyclized.

**21.** The method of paragraph **13** wherein the peptide is dimerized.

**22.** A method for treating a patient having a disorder characterized by a deficiency of erythropoietin or a low or defective red blood cell population comprising administering to the patient a therapeutically effective amount of a peptide of 10 to 40 amino acid residues in length that binds to erythropoietin receptor and comprises a sequence of amino acids $X_3X_4X_5GPX_6TWX_7X_8$ (SEQ ID NO:) where each amino acid is indicated by standard one letter abbreviation; $X_6$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**23.** The method of paragraph **22** wherein the disorder is end-stage renal failure or dialysis; anemia associated with AIDS, autoimmune disease, or malignancies; beta-thalassemia; cystic fibrosis; early anemia of prematurity; anemia associated with chronic inflammatory diseases; spinal cord injury; acute blood loss; aging; and neoplastic disease states accompanied by abnormal erythropoiesis.

**24.** The method of paragraph **22** wherein the peptide comprises a sequence of amino acids $YX_2X_3X_4X_5GPX_6TWX_7X_8$ where each amino acid is indicated by standard one letter abbreviation; each $X_2$ and $X_6$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

**25.** The method of paragraph **24** wherein the peptide comprises a sequence of amino acids $X_1YX_2X_3X_4X_5GPX_6TWX_7X_8X_9X_{10}X_{11}$, where each amino acid is indicated by standard one letter abbreviation; each $X_1$ $X_2$, $X_6$, $X_9$, $X_{10}$, and $X_{11}$ is independently selected from any one of the 20 genetically coded L-amino acids;

$X_3$ is C; $X_4$ is R, H, L, or W; $X_5$ is M, F, or I; $X_7$ is D, E, I, L, or V; and $X_8$ is C.

**26.** The method of paragraph **25** wherein $X_4$ is R or H; $X_5$ is F or M; $X_6$ is I, L, T, M, or V; $X_7$ is D or V; $X_9$ is G, K, L, Q, R, S, or T; and $X_{10}$ is A, G, P, R, or Y.

**27.** The method of paragraph **26** wherein $X_1$ is D, E, L, N, S, T, or V; $X_2$ is A, H, K, L, M, S, or T; $X_4$ is R or H; $X_9$ is K, R, S, or T; and $X_{10}$ is P.

**28.** The method of paragraph **22** wherein the peptide is selected from the group consisting of:

GGLYLCRFGPVTWDCGYKGG;
GGTYSCHFGPLTWVCKPQGG;
GGTYSCHFGPLTWVCKPQ;
GGDYHCRMGPLTWVCKPLGG;
VGNYMCHFGPITWVCRPGGG;
GGNYMCHFGPITWVCRPGGG;
GGVYACRMGPITWVCSPLGG;
VGNYMAHMGPITWVCRPGG;
GGPHHVYACRMGPLTWIC;
GGTYSCHFGPLTWVCKPQ;

GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
SCHFGPLTWVCK;
GGTYSCFGPLTWVCKPQGG;
TYSCHFGPLTWVCKPQGG;
YSCHFGPLTWVC;
GGTYSCHFGPLTFVCKPQGG; and
HFGPLTWV.

**29.** The method of paragraph **22** wherein the peptide is cyclized.

**30.** The method of paragraph **22** wherein the peptide is dimerized.

## Claims

1. A peptide of 10 to 40 amino acid residues in length that binds to and activates erythropoietin receptor or otherwise behaves as an erythropoietin agonist, and comprises a sequence of amino acids $X_3X_4X_5GPX_6TWX_7X_8$, optionally cyclized or dimerized, where each amino acid is indicated by standard one letter abbreviation; $X_6$ is independently selected from any one of the 20 genetically coded L-amino acids; $X_3$ is C, A, α-amino-γ-bromobutyric acid, or Hoc, where Hoc is homocysteine; $X_4$ is R, H, L, or W; $X_5$ is M, F, or I; $X_7$ is D, E, I, L, or V; and $X_8$ is C, A, α-amino-γ-bromobutyric acid, or Hoc, where hoc is homocysteine, provided that either $X_3$ or $X_8$ is C or Hoc.

2. A peptide of 10 to 40 amino acid residues in length that binds to erythropoietin receptor and comprises a sequence of amino acids $X_3X_4X_5GPX_6TWX_7X_8$, optionally cyclized or dimerized, where each amino acid is indicated by standard one letter abbreviation; $X_6$ is independently selected from any one of the 20 genetically coded L-amino acids; $X_3$ is C; $X_4$ is R, H, L, or W; $X_5$ is M, F, or I; $X_7$ is D, E, I, L, or V; and $X_8$ is C.

3. A peptide of claim 1 or claim 2 that comprises either:

   (a) a sequence of amino acids $YX_2X_3X_4X_5GPX_6TWX_7X_8$ where each amino acid is indicated by standard one letter abbreviation; $X_2$ is independently selected from any one of the 20 genetically coded L-amino acids; $X_3$, $X_4$, $X_5$, $X_6$, $X_7$ and $X_8$ are as defined in claim 1 or claim 2

   (b) a sequence of amino acids $X_1YX_2X_3X_4X_5GPX_6TWX_7X_8X_9X_{10}X_{11}$, where each amino acid is indicated by standard one letter abbreviation; each of $X_1$, $X_2$, $X_9$, $X_{10}$, and $X_{11}$ is independently selected from any one of the 20 genetically coded L-amino acids; $X_3$, $X_4$, $X_5$, $X_6$, $X_7$ and $X_8$ are as defined in claim 1 or claim 2.

4. A peptide of claim 3 (b) wherein $X_4$ is R or H; $X_5$ is F or M; $X_6$ is I, L, T, M, E, or V; $X_7$ is D or V; Xg is G, K, L, Q, R, S, or T; and $X_{10}$ is A, G, P, R, or Y, preferably wherein $X_1$ is D, E, L, N, S, T, or V; $X_2$ is A, H, K, L, M, S, or T; $X_4$ is R or H; $X_9$ is K, R, S, or T; and $X_{10}$ is P.

5. A peptide of claim 1 or claim 2 that is selected from the group consisting of:

GGLYLCRFGPVTWDCGYKGG;
GGTYSCHFGPLTWVCKPQGG;
GGTYSCHFGPLTWVCKPQ;
GGDYHCRMGPLTWVCKPLGG;
VGNYMCHFGPITWVCRPGGG;
GGNYMCHFGPITWVCRPGGG;
GGVYACRMGPITWVCSPLGG;
VGNYMAHMGPITWVCRPGG;
GGPHHVYACRMGPLTWIC;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
SCHFGPLTWVCK;
GGTYSCFGPLTWVCKPQGG;
TYSCHFGPLTWVCKPQGG;
YSCHFGPLTWVC;
GGTYSCHFGPLTFVCKPQGG; and
HFGPLTWV.

**6.** A peptide of claim 1 or claim 2 wherein said sequence of amino acids is cyclized, selected from the group consisting of:

GGTYSCHFGPLTWVCKPQGG;
GGTYSCHFGPLTWVCKPQ;
GGLYACHMGPMTWVCQPLRG;
TIAQYICYMGPETWECRPSPKA;
YSCHFGPLTWVCK;
YCHFGPLTWVC;
Ac-GGTYSCHFGPLTWVCKPQGG;
GGCRIGPITWVCGG;
LGRKYSCHFGPLTWVCQPAKKD;
GGTASCHFGPLTWVCKPQGG;
GGNYYCRFGPITFECHPTGG;
GGEYLCRMGPMTWVCTPVGG;
GGLYTCRMGPITWVCLPAGG;
GGTTSCHFGPLTWVCKPQGG;
GGTFSCHFGPLTWVCKPQGG;
GGTYSCHFGALTWVCKPQGG;
GGTYSCHFGPLAWVCKPQGG;
GGTYSCHFAPLTWVCKPQGG;
GGTYSCHFGPATWVCKPQGG;
GGTYSCHFGPLTAVCKPQGG;
GGTYSCHFGPLTFVCKPQGG;
TYSCHFGPLTWVCKPQ;
YSCHFGPLTWVCKP;
YSCHFGALTWVCK;
SCHFGPLTWVCK;
GGTYSEHFGPLTWVKKPQGG;
GGTYSCFGPLTWVCKPQGG;
TYSCHFGPLTWVCKPQGG;
YSCHFGPLTWVC;
GGTYSCHFGPLTFVCKPQGG; and
GGTYSEHFGPLTWVKKPQGG

7. A peptide of claim 1 or claim 2 wherein said sequence of amino acids is dimerized, which peptide comprises the following amino acid sequence:

GGTYSCHFGPLTWVCKPQGG
GGTYSCHFGPLTWVCKPQGG.

8. A peptide as claimed in any preceding claim conjugated to a further compound.

9. A peptide as claimed in claim 8 wherein the further compound binds to an erythropoietin receptor.

10. A peptide as claimed in claim 8 wherein the further compound is a nonproteinaceous polymer.

11. A peptide as claimed in claim 8 wherein the further compound is a protein.

12. A pharmaceutical composition comprising a compound of any one of claims 1 to 11 in combination with a pharmaceutically acceptable carrier.

**13.** A compound of any one of claims 1 to 11 for use as a pharmaceutical.

**14.** The use of a compound of any one of claims 1 to 11 in the manufacture of a medicament for treating a patient having a disorder **characterized by** a deficiency of erythropoietin or a low or defective red blood cell population.

**15.** A use of claim 14 wherein the medicament is for treating a patient having a disorder which is end-stage renal failure or dialysis; anemia associated with AIDS, autoimmune disease, or malignancies; beta-thalassemia; cystic fibrosis; early anemia of prematurity; anemia associated with chronic inflammatory diseases; spinal cord injury; acute blood loss; aging; and neoplastic disease states accompanies by abnormal erythropoiesis.

RIGPITWV MUTAGENESIS OLIGO

70/10/10/10

C TCT CAC TCC GGA GGC NNK NNK NNK TGT cgK atK ggK ccK atK acK

tgK gtK TGT NNK NNK NNK GGA GGC GGG GGT AGC ACT GTT GAA

AGT TGT

*FIG. 1.*

| | |
|---|---|
| GGTYRCSMGPMTWVCLPMGG | SEQ ID NO: |
| GGMYSCRMGPMTWVCGPSGG | SEQ ID NO: |
| GGWAWCRMGPITWVCSAHGG | SEQ ID NO: |
| GGMYSCRMGPMTWVCIPYGG | SEQ ID NO: |
| GGEYKCYMGPITWVCKPEGG | SEQ ID NO: |
| GGDYTCRMGPMTWICTATGG | SEQ ID NO: |
| GGNYLCRFGPGTWDCTGFRG | SEQ ID NO: |
| GGNYVCRMGPITWICTPAGG | SEQ ID NO: |
| GGKDVCRMGPITWDCRSTGG | SEQ ID NO: |
| GGSYLCRMGPTTWLCTAQRGGGN | SEQ ID NO: |
| GGNYLCRMGPATWVCGRMGG | SEQ ID NO: |
| GGEYKCRMGPLTWVCQYAGG | SEQ ID NO: |
| GGDYTCRMGPMTWICTATRG | SEQ ID NO: |
| GGVYVCRMGPLTWECTASGG | SEQ ID NO: |
| GGEYSCRMGPMTWVCSPTGG | SEQ ID NO: |
| GGEYLCRMGPITWVCERYGG | SEQ ID NO: |
| GGNYICRMGPMTWVCTAHGG | SEQ ID NO: |
| GGDYLCRMGPATWVCGRMGG | SEQ ID NO: |
| GGLYLCRFGPVTWDCGYKGG | SEQ ID NO: |
| GGLYSCRMGPITWVCTKAGG | SEQ ID NO: |
| GGGYHCRMGPMTWVCRPVGG | SEQ ID NO: |
| GGTYSCHFGPLTWVCKPQGG | SEQ ID NO: |
| GGIYKCLMGPLTWVCTPDGG | SEQ ID NO: |
| GGLYSCLMGPITWLCKPKGG | SEQ ID NO: |
| GGDYHCRMGPLTWVCKPLGG | SEQ ID NO: |
| GGDYSCRMGPTTWVCTPPGG | SEQ ID NO: |
| GGDYWCRMGPSTWECNAHGG | SEQ ID NO: |
| GGKYLCSFGPITWVCARYGG | SEQ ID NO: |
| GGLYKCRLGPITWVCSPLGG | SEQ ID NO: |
| GGSYTCRFGPETWVCRPNGG | SEQ ID NO: |
| GGSYSCRMGPITWVCKPGGG | SEQ ID NO: |
| GGSYTCRMGPITWVCLPAGG | SEQ ID NO: |
| GGLYECRMGPMTWVCRPGGG | SEQ ID NO: |

*FIG. 2-1.*

| | |
|---|---|
| GGDYTCRMGPITWICTKAGG | SEQ ID NO: |
| GGVYSCRMGPTTWECNRYVG | SEQ ID NO: |
| GGAYLCHMGPITWVCRPQGGG | SEQ ID NO: |
| GGEYSCRMGPNTWVCRPVGG | SEQ ID NO: |
| GGLYLCRMGPVTWECQPRGG | SEQ ID NO: |
| GGLYTCRMGPITWVCLLPGG | SEQ ID NO: |
| GGLYTCRMGPVTWVCTGAGG | SEQ ID NO: |
| GGVYKCRMGPLTWECRPTGG | SEQ ID NO: |
| GGDYNCRFGPLTWVCKPSGG | SEQ ID NO: |
| GGSYLCRFGPTTWLCSSAGG | SEQ ID NO: |
| GGSYLCRMGPTTWVCTRMGG | SEQ ID NO: |
| GGSYLCRFGPTTWLCTQRGG | SEQ ID NO: |
| GGWVTCRMGPITWVCGVHGG | SEQ ID NO: |
| GGQLLCGIGPITWVCRWVGG | SEQ ID NO: |
| GGKYSCFMGPTTWVCSPVGRGV | SEQ ID NO: |
| GGWVYCRIGPITWVCDTNGG | SEQ ID NO: |
| GGMYYCRMGPMTWVCKGAGG | SEQ ID NO: |
| GGTTQCWIGPITWVCRARGG | SEQ ID NO: |
| GGPYHCRMGPITWVCGPVGG | SEQ ID NO: |
| GGEYRCRMGPISWVCSPQGG | SEQ ID NO: |
| GGNYTCRFGPLTWECTPQGGGA | SEQ ID NO: |
| GGSWDCRIGPITWVCKWSGG | SEQ ID NO: |
| VGNYMCHFGPITWVCRPGGG | SEQ ID NO: |
| GGLYLCRMGPQTWMCQPGGG | SEQ ID NO: |
| GGDYVCRMGPMTWVCAPYGR | SEQ ID NO: |
| GGWYSCLMGPMTWVCKAHRG | SEQ ID NO: |
| GGKYYCWMGPMTWVCSPAGG | SEQ ID NO: |
| GGYVMCRIGPITWVCDIPGG | SEQ ID NO: |
| GSCLQCCIGPITWVCRHAGG | SEQ ID NO: |
| GGNYFCRMGPITWVCQRSVG | SEQ ID NO: |
| GGEYICRMGPLTWECKRTGG | SEQ ID NO: |
| GGLYACRMGPITWVCKYMAG | SEQ ID NO: |
| GGQYLCTFGPITWLCRGAGG | SEQ ID NO: |
| GGVYACRMGPTTWVCSPLGG | SEQ ID NO: |
| GGYTTCRMGPITWVCSAHGG | SEQ ID NO: |
| GGTYKCWMGPMTWVCRPVGG | SEQ ID NO: |
| GGNYYCRFGPITRECHPTGG | SEQ ID NO: |
| GGLYACHMGPMTWVCQPLRG | SEQ ID NO: |
| GGEYLCRMGPMTWVCTPVGG | SEQ ID NO: |
| GGLYTCRMGPITWVCLPAGG | SEQ ID NO: |
| GGLYTCRMGPITWVCLPAGG | SEQ ID NO: |

*FIG. 2-2.*

MUTAGENESIS LIBRARY ON2598

GGXXXXYXCR IGPITWVCXXXXXX (G4S) 4 - pIIIV

*FIG. 3.*

ON 3007
(NNK)$_{10}$ **Tyr** (NNK) Cys (NNK)$_2$ **Gly Pro** (NNK) **Thr Trp** (NNK) **Cys (Gly)$_4$ Ser**-pIII/pVIII

*FIG. 4A.*

ON 3016
**Gly Gly** (NNK)$_4$**Tyr Cys** (NNK)$_2$ **Gly Pro** (NNK) **Thr Trp** (NNK) **Cys** (NNK)$_5$ **(Gly)$_4$Ser - pIII**/pVIII

*FIG. 4B.*

ON 3017
**Gly Gly Tyr** (NNK) **Cys** (NNK)$_2$ **Gly Pro** (NNK) **Thr Trp** (NNK) **Cys** (NNK)$_{10}$ - **(Gly)$_4$ Ser - pIII**/pVIII

NNK = RANDOM AMINO ACID RESIDUE.
RESIDUES IN **BOLD** ARE FIXED.

*FIG. 4C.*

AF11157
ggTYSCHFGPLTWVCKPQgg
↓
-X$_4$YXCH FGPLTWVCX$_6$ (C-TERMINUS)

*FIG. 5.*

ON3236

- $X_4$YXCXXGPETWECX$_6$

(C-terminus

Y-C-G-P-T-W-C fixed

**E** 91:3:3:3/91:3:3:3/K

**X** NNK

FIGURE 6

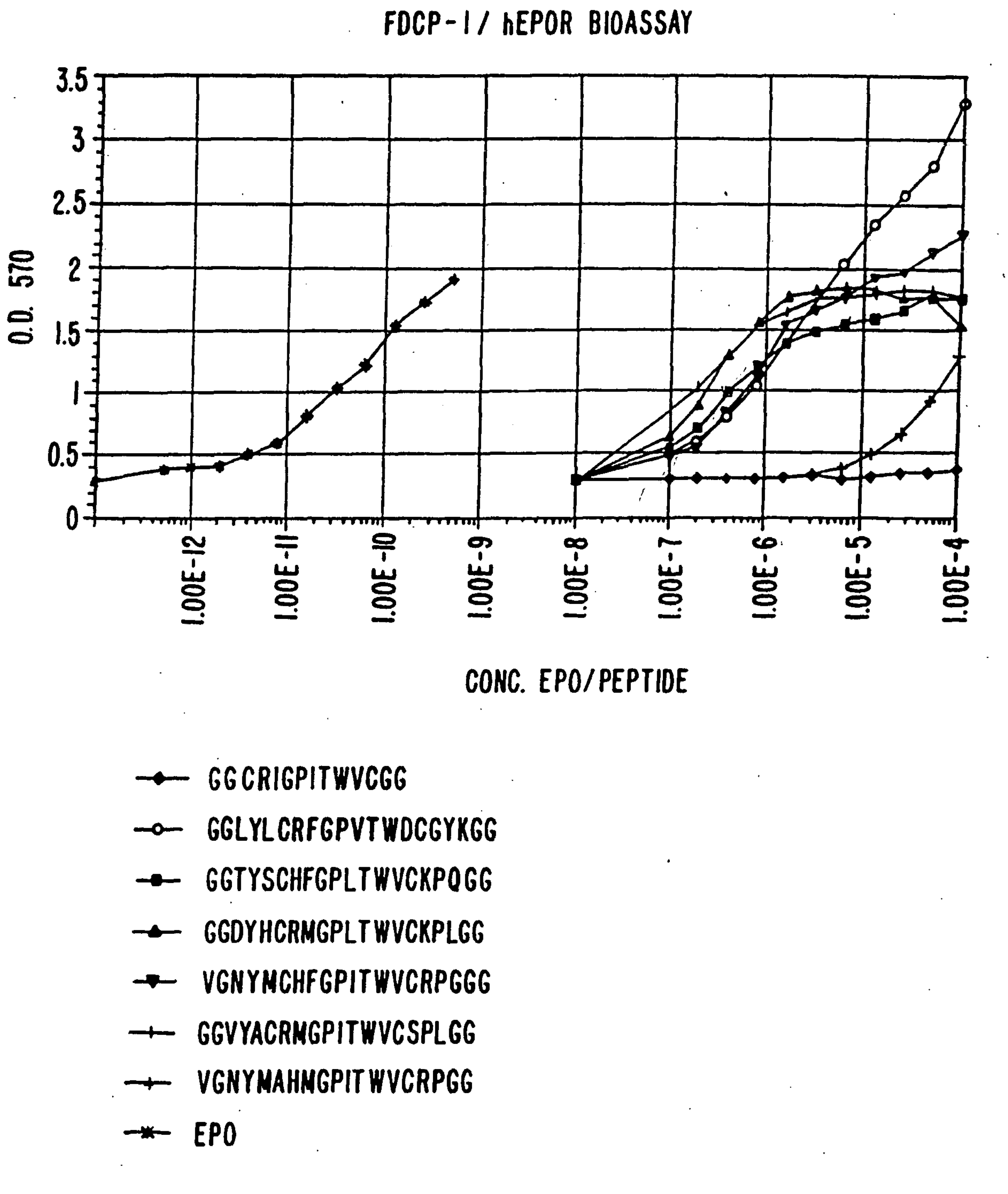
FDCP-1/ hEPOR BIOASSAY
O.D. 570
3.5
3
2.5
2
1.5
1
0.5
0
1.00E-12
1.00E-11
1.00E-10
1.00E-9
1.00E-8
1.00E-7
1.00E-6
1.00E-5
1.00E-4
CONC. EPO/PEPTIDE
GGCRIGPITWVCGG
GGLYLCRFGPVTWDCGYKGG
GGTYSCHFGPLTWVCKPQGG
GGDYHCRMGPLTWVCKPLGG
VGNYMCHFGPITWVCRPGGG
GGVYACRMGPITWVCSPLGG
VGNYMAHMGPITWVCRPGG
EPO

FIG. 7

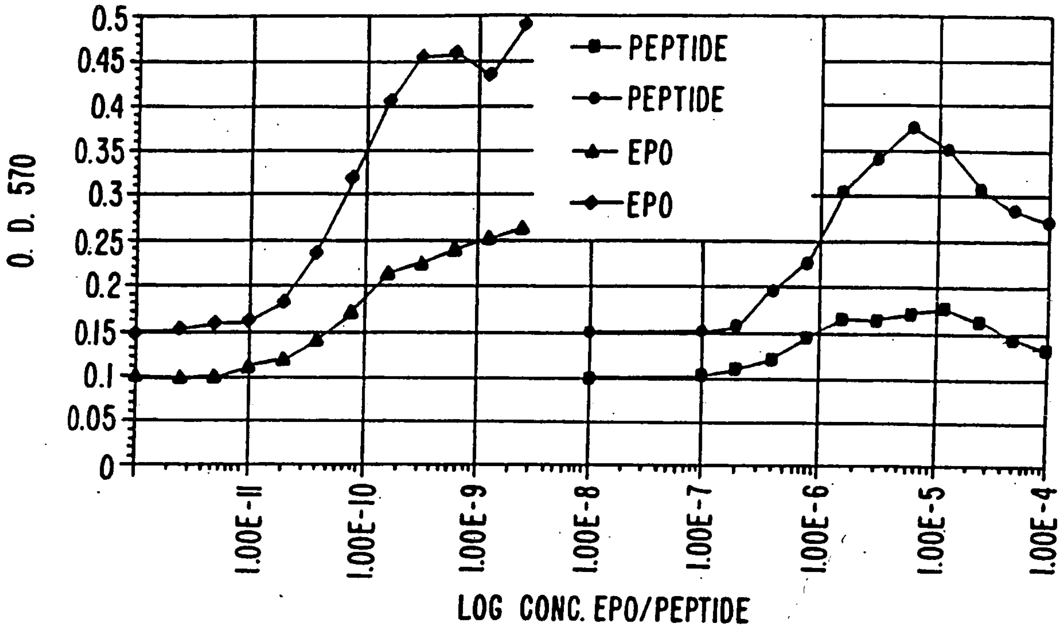
O. D. 570
0.5
0.45
0.4
0.35
0.3
0.25
0.2
0.15
0.1
0.05
0
PEPTIDE
PEPTIDE
EPO
EPO
1.00E-11
1.00E-10
1.00E-9
1.00E-8
1.00E-7
1.00E-6
1.00E-5
1.00E-4
LOG CONC. EPO/PEPTIDE

FIG. 8

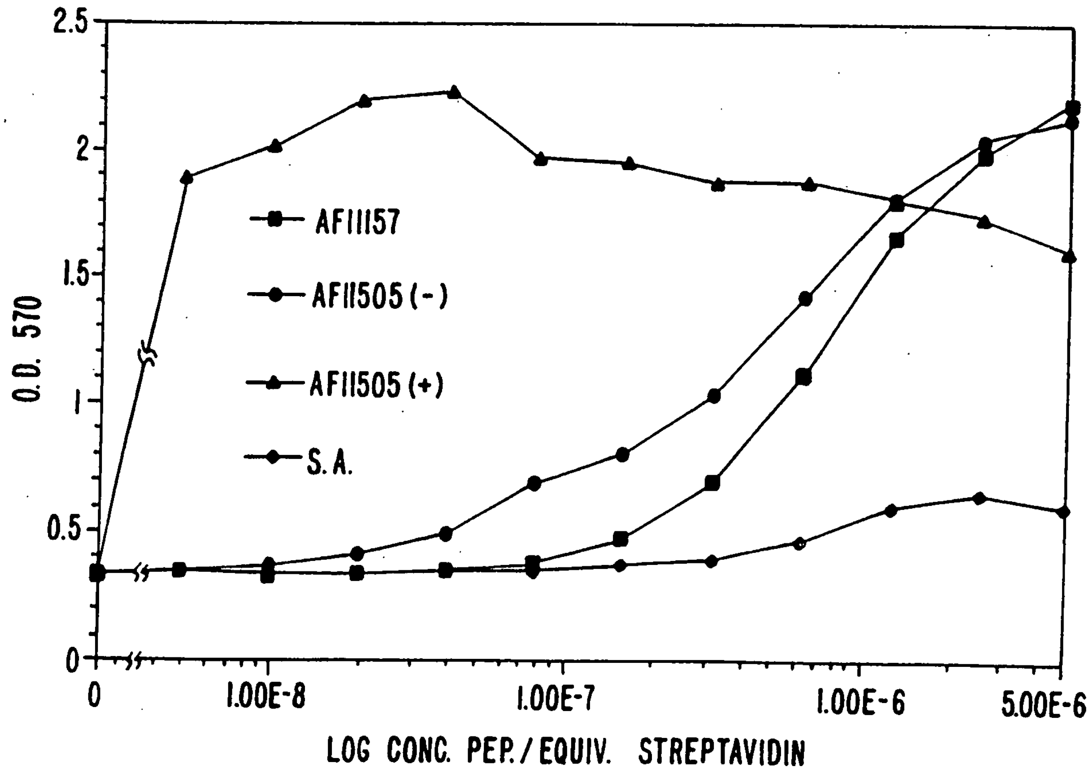
O.D. 570
2.5
2
1.5
1
0.5
0
AF11157
AF11505 (-)
AF11505 (+)
S. A.
0
1.00E-8
1.00E-7
1.00E-6
5.00E-6
LOG CONC. PEP. / EQUIV. STREPTAVIDIN

FIG. 10

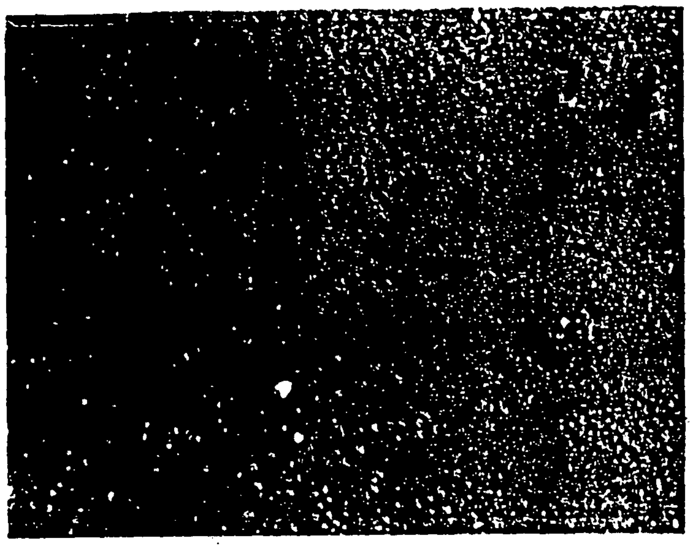

Figure 9A

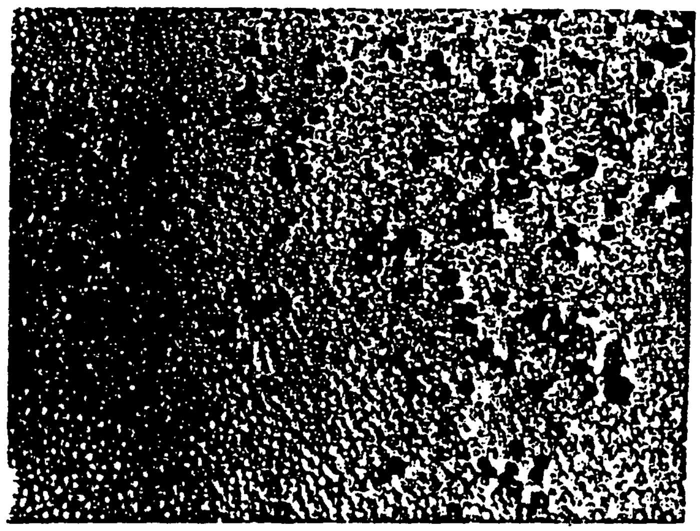

Figure 9B

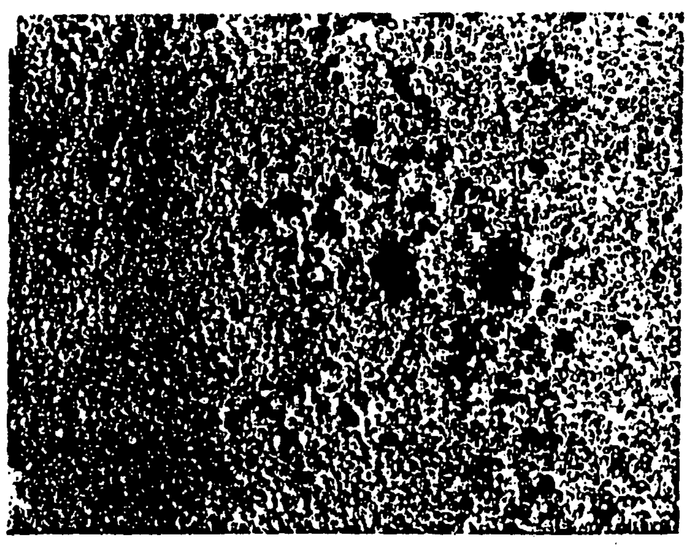

Figure 9C

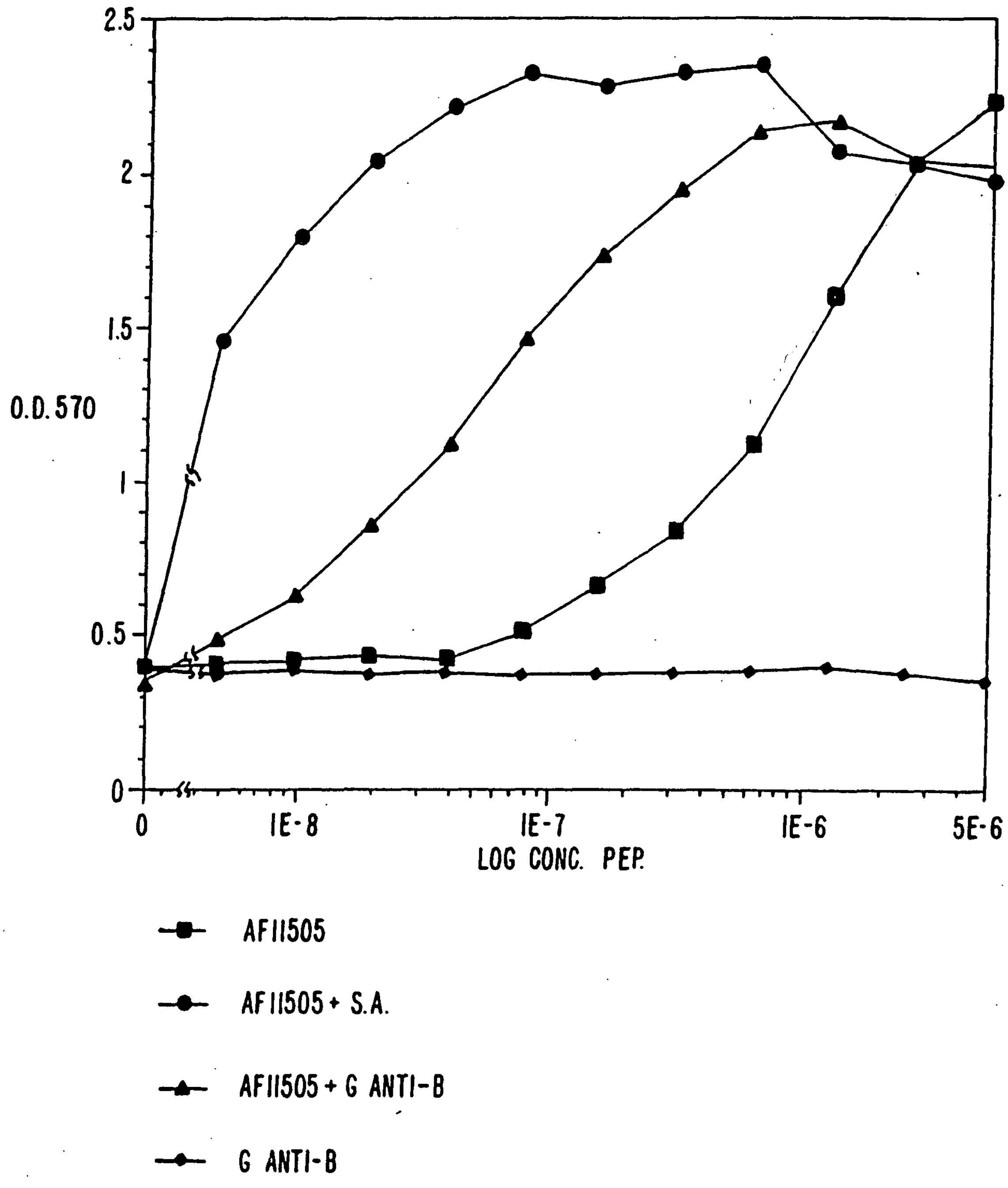
2.5
2
1.5
O.D.570
1
0.5
0
0
IE-8
IE-7
IE-6
5E-6
LOG CONC. PEP.
AF11505
AF11505 + S.A.
AF11505 + G ANTI-B
G ANTI-B

FIG. 11

## SYNTHETIC SCHEME FOR DIMER PREPARATION

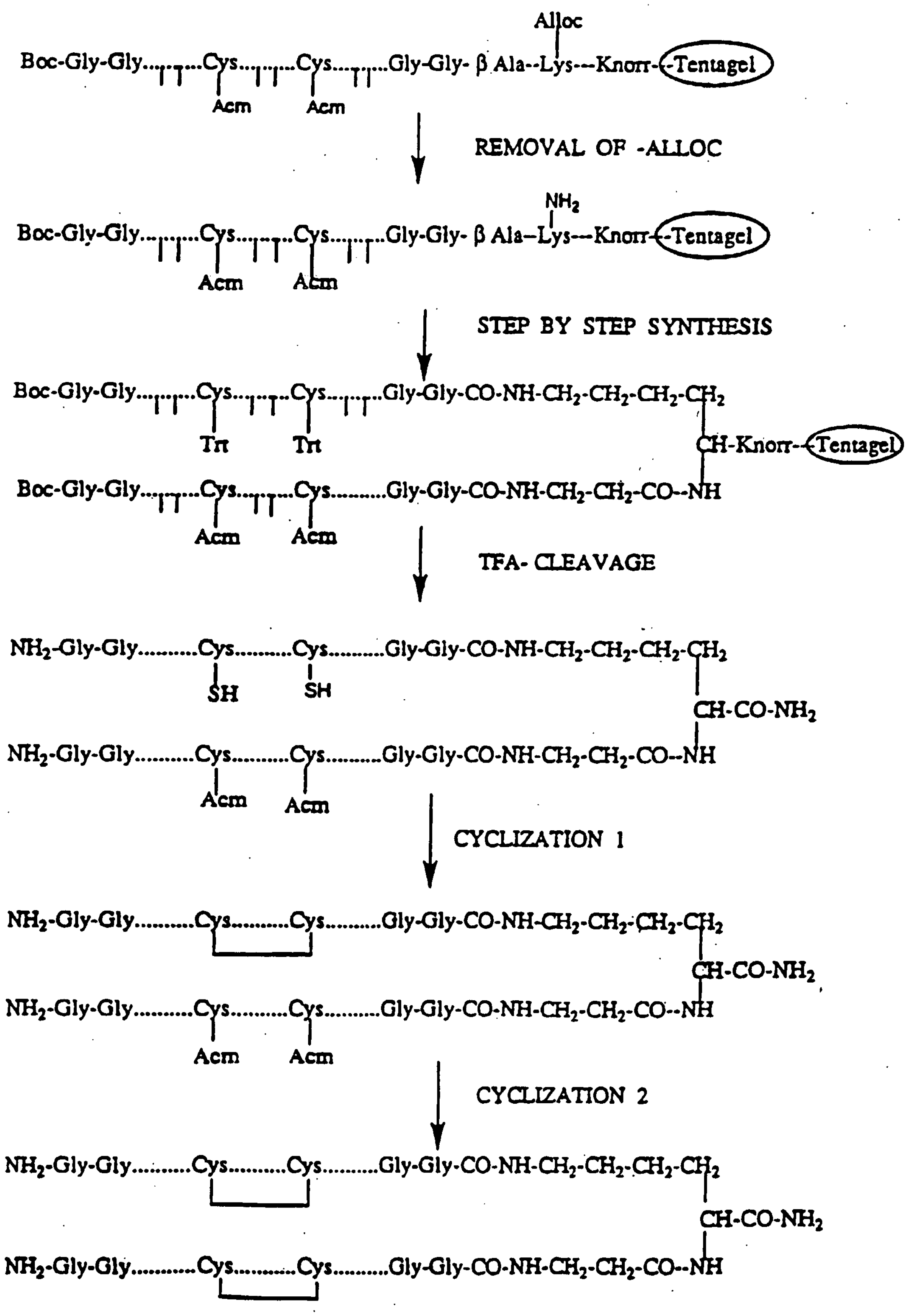

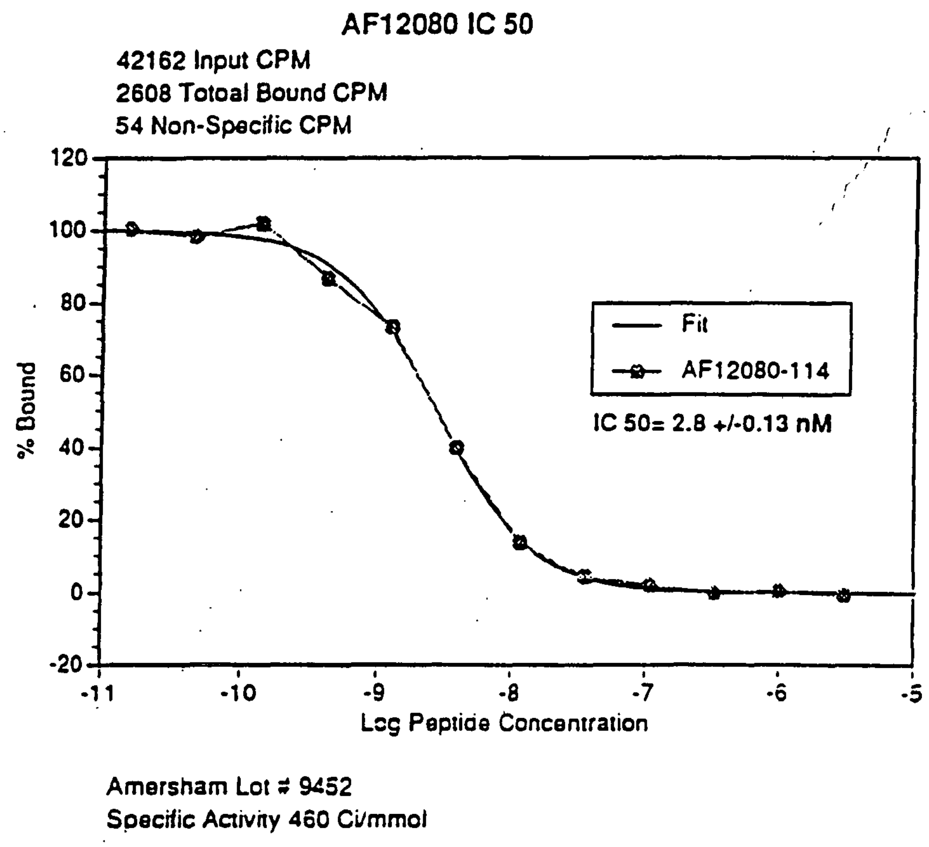
AF12080 IC 50
42162 Input CPM
2608 Totoal Bound CPM
54 Non-Specific CPM
120
100
80
60
40
20
0
-20
% Bound
-11
-10
-9
-8
-7
-6
-5
Log Peptide Concentration
Fit
AF12080-114
IC 50= 2.8 +/-0.13 nM
Amersham Lot # 9452
Specific Activity 460 Ci/mmol

FIGURE 13

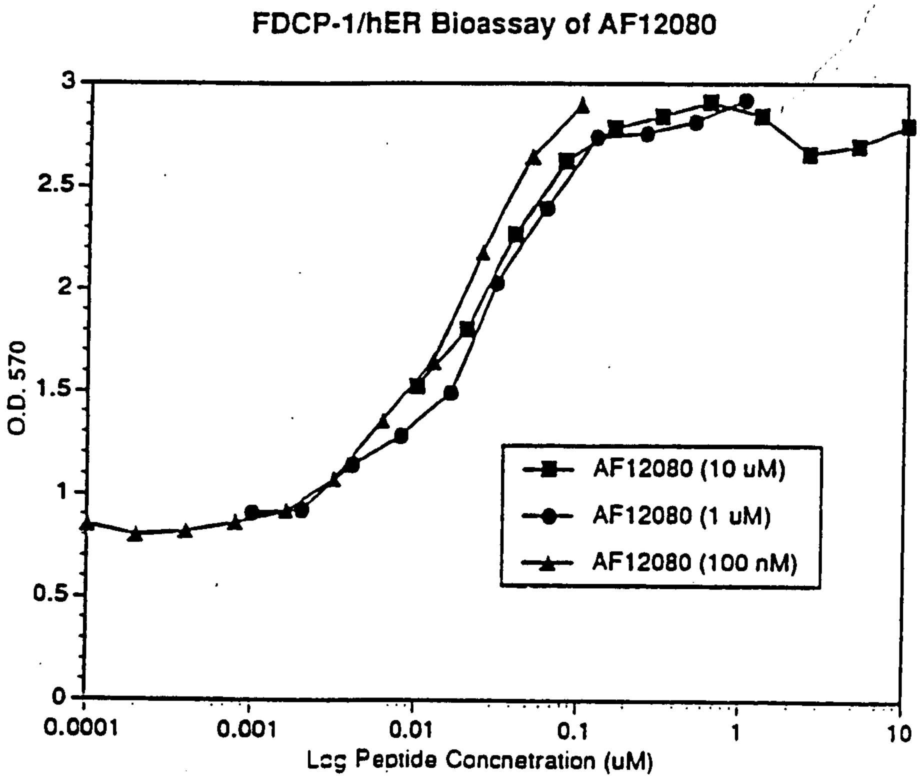
FDCP-1/hER Bioassay of AF12080
O.D. 570
3
2.5
2
1.5
1
0.5
0
0.0001
0.001
0.01
0.1
1
10
Log Peptide Concnetration (uM)
AF12080 (10 uM)
AF12080 (1 uM)
AF12080 (100 nM)

FIGURE 14

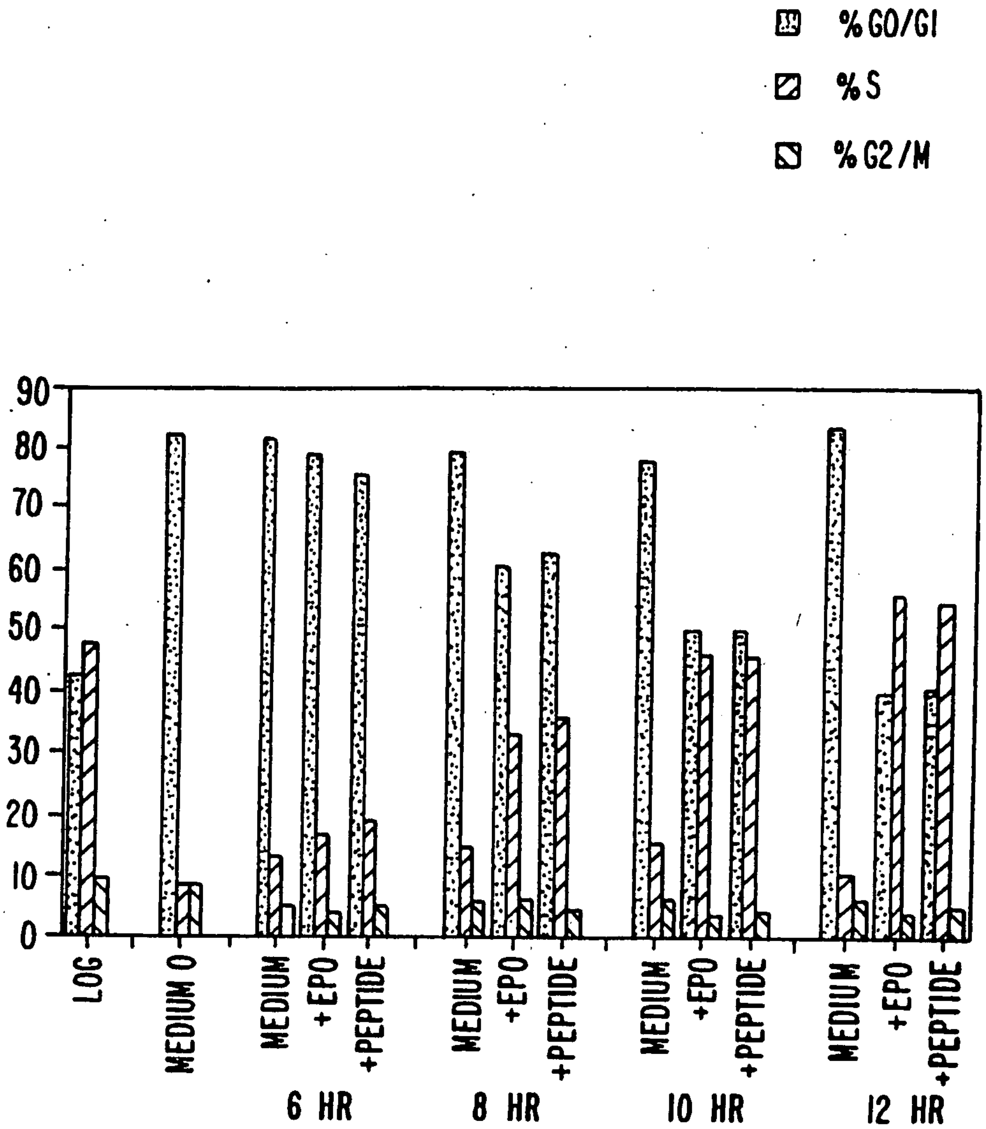
%G0/G1
%S
%G2/M
90
80
70
60
50
40
30
20
10
0
LOG
MEDIUM 0
MEDIUM
+EPO
+PEPTIDE
6 HR
MEDIUM
+EPO
+PEPTIDE
8 HR
MEDIUM
+EPO
+PEPTIDE
10 HR
MEDIUM
+EPO
+PEPTIDE
12 HR

FIG. 15

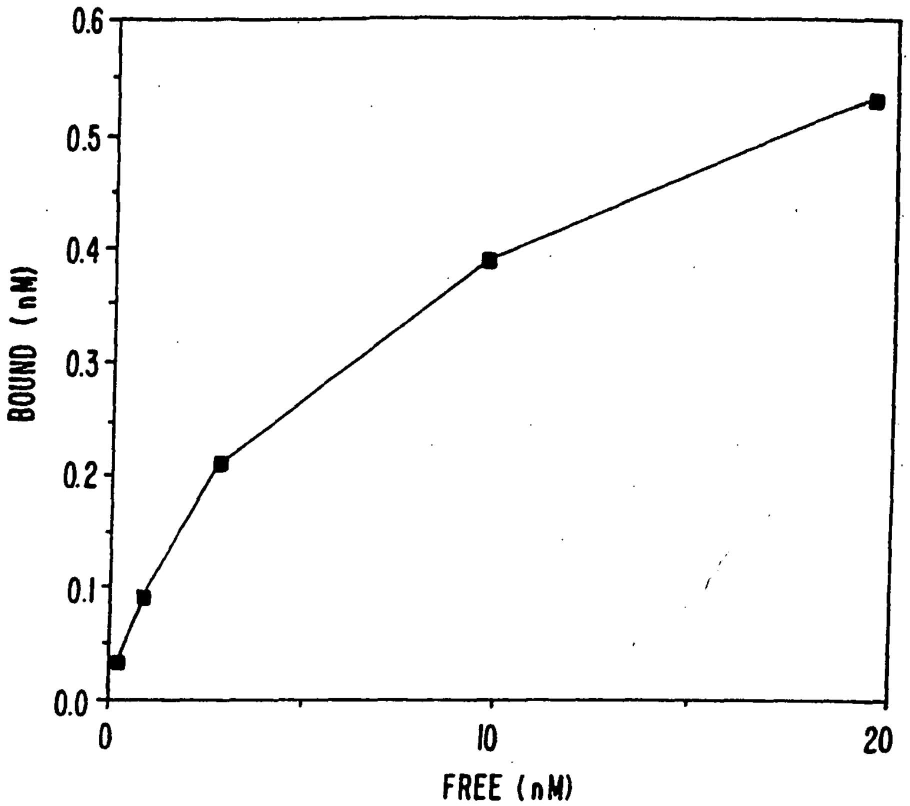


FIG. 16A

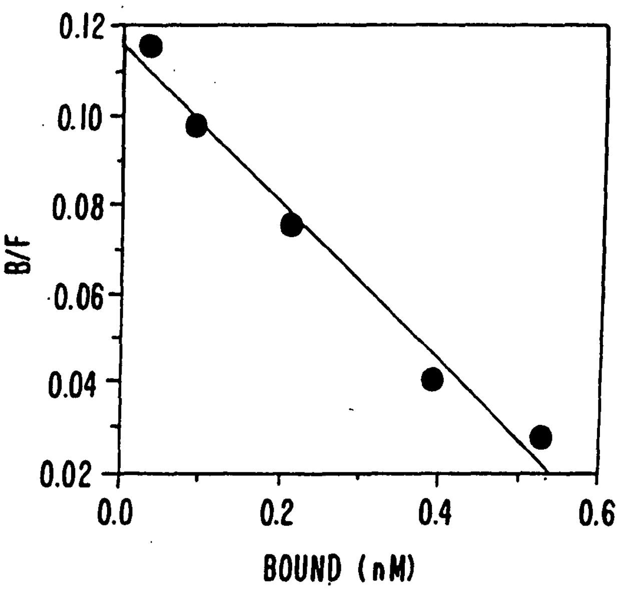


FIG. 16B

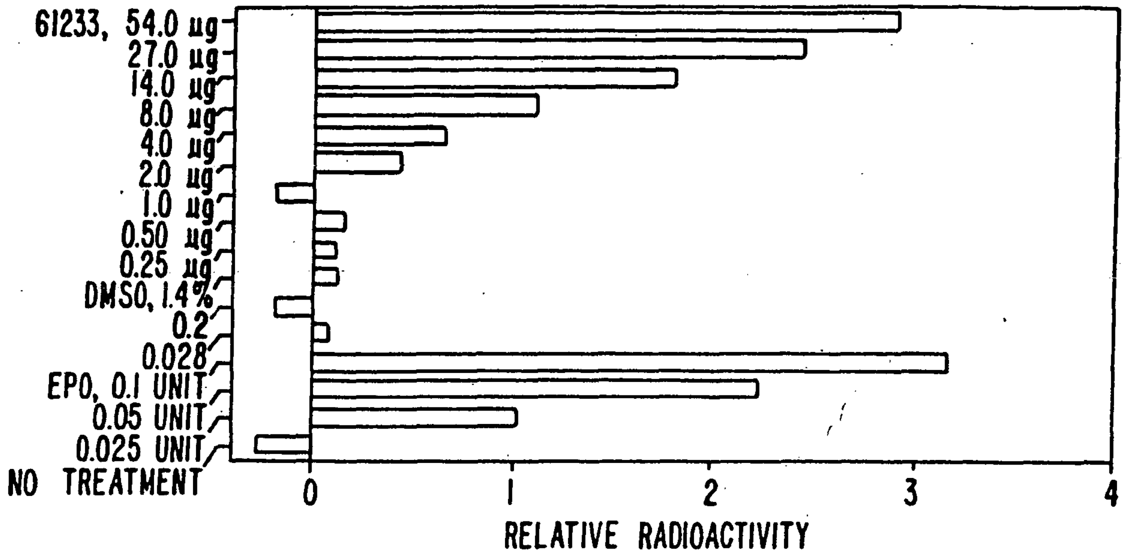


*FIG.* 17A

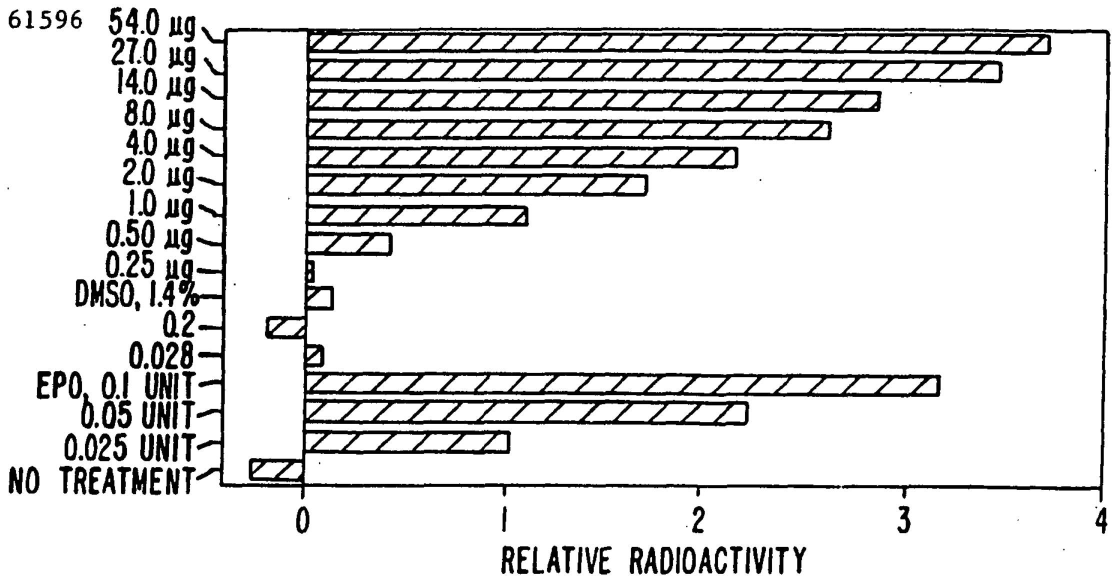


*FIG.* 17B

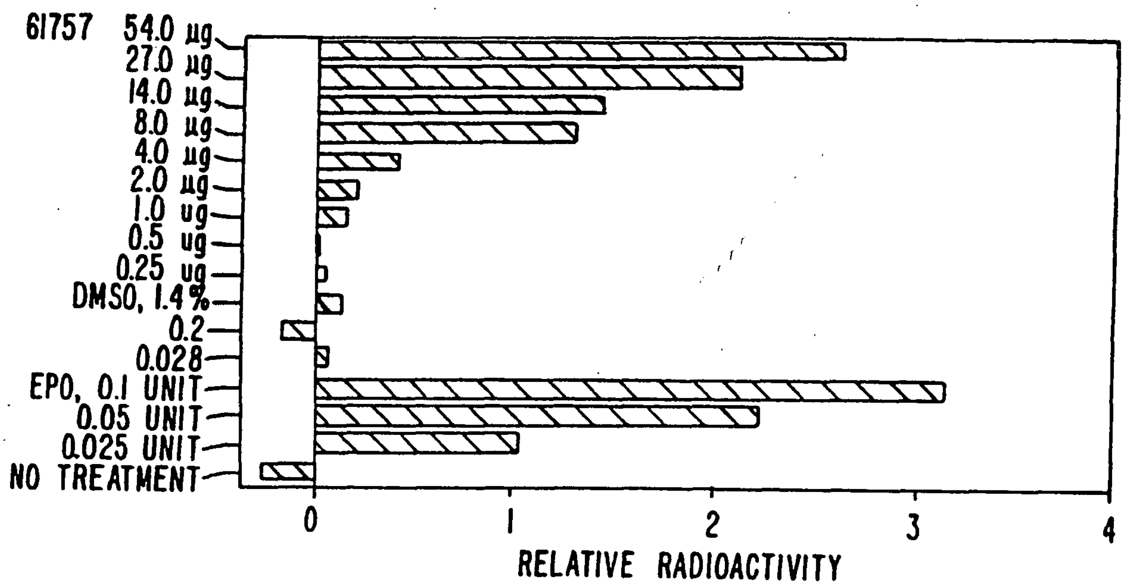
61757 54.0 µg
27.0 µg
14.0 µg
8.0 µg
4.0 µg
2.0 µg
1.0 ug
0.5 ug
0.25 ug
DMSO, 1.4%
0.2
0.028
EPO, 0.1 UNIT
0.05 UNIT
0.025 UNIT
NO TREATMENT
0
1
2
3
4
RELATIVE RADIOACTIVITY

FIG. 17C

FIGURE 18A

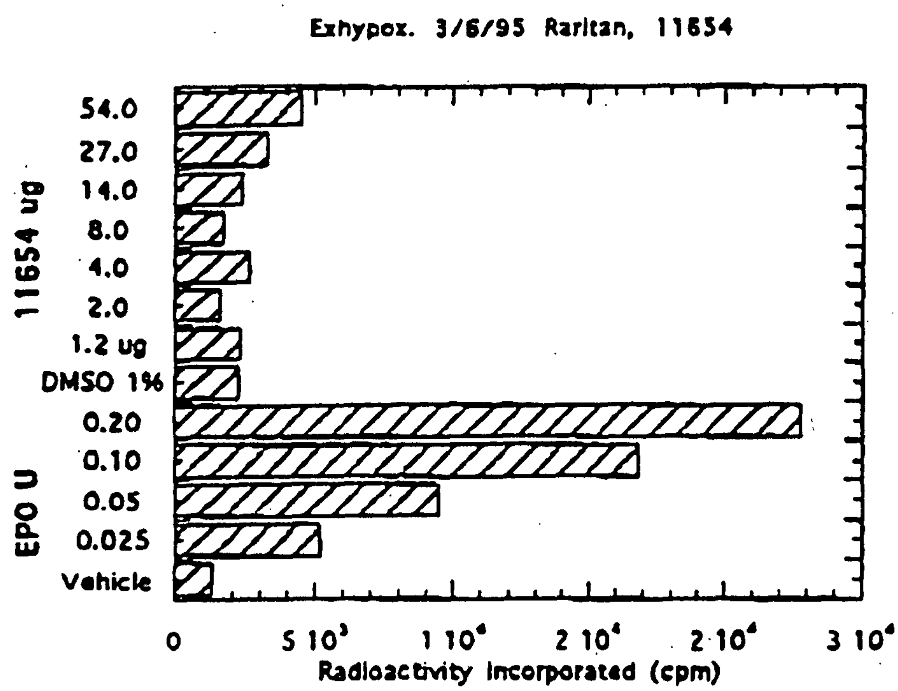
Exhypox. 3/6/95 Raritan, 11654
11654 ug
54.0
27.0
14.0
8.0
4.0
2.0
1.2 ug
DMSO 1%
EPO U
0.20
0.10
0.05
0.025
Vehicle
0
5 10³
1 10⁴
2 10⁴
2 10⁴
3 10⁴
Radioactivity Incorporated (cpm)

FIGURE 18B

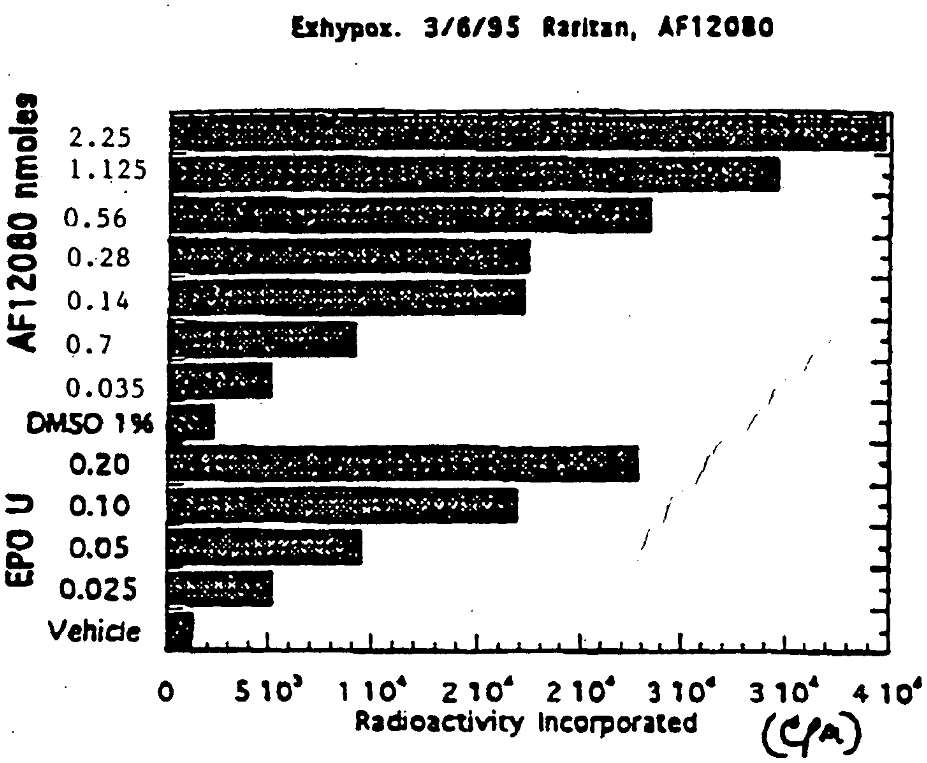
Exhypox. 3/6/95 Raritan, AF12080
AF12080 nmoles
2.25
1.125
0.56
0.28
0.14
0.7
0.035
DMSO 1%
0.20
EPO U
0.10
0.05
0.025
Vehicle
0
5 10³
1 10⁴
2 10⁴
2 10⁴
3 10⁴
3 10⁴
4 10⁴
Radioactivity Incorporated
(C/A)

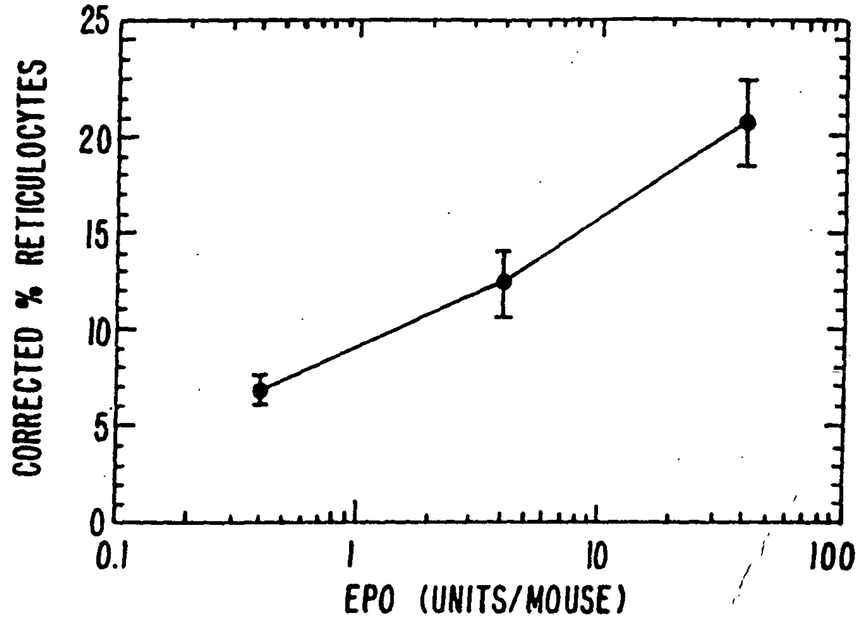


Figure 19A

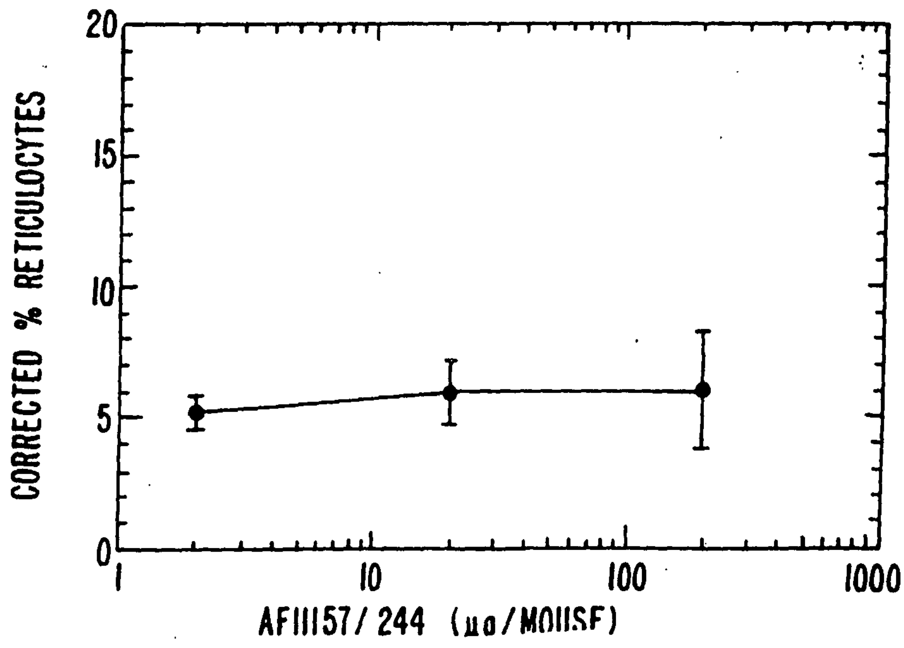


Figure 19B

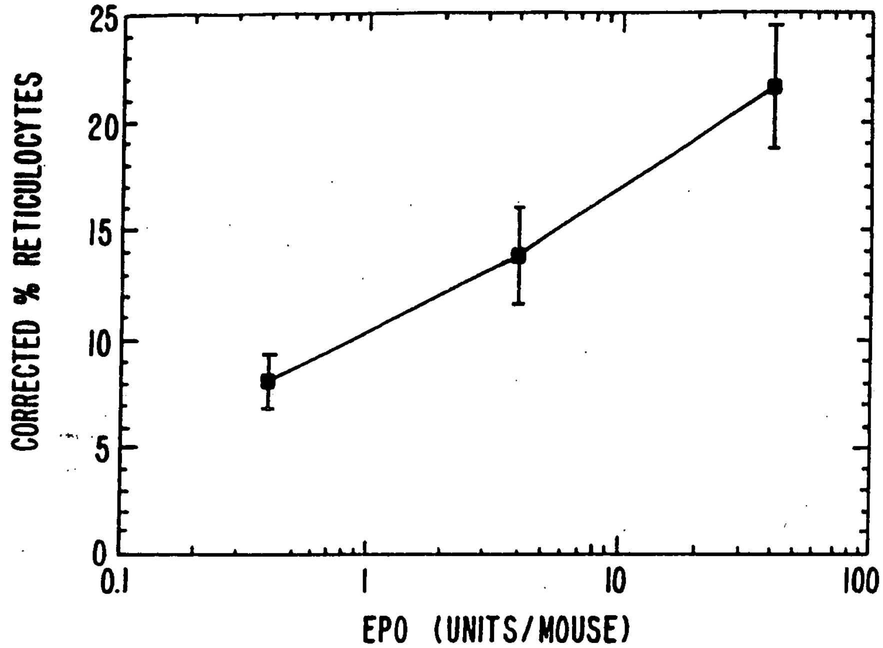


Figure 20A

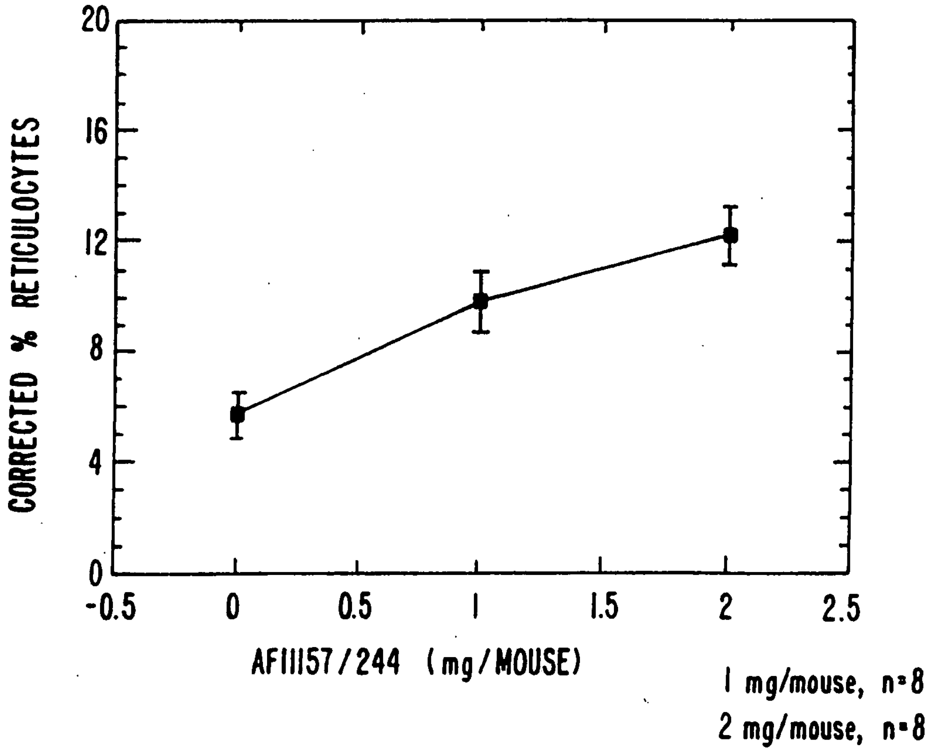


Figure 20B